# EUROPEAN PATENT APPLICATION

(11) **EP 4 548 932 A1**
(43) Date of publication of application: **07.05.2025**
(21) Application number: 23831562.6
(22) Date of filing: 29.06.2023
(51) Int. Cl.: A61K 39/395, A61K 31/5513, A61K 47/68, A61P 35/00, A61P 35/02, C07D 519/00, C07K 16/28, C12N 15/13

(54) **PHARMACEUTICAL COMPOSITION FOR TREATING AND/OR PREVENTING CANCER**

(30) Priority: 30.06.2022 JP 2022105374
(71) Applicant: Toray Industries, Inc., Tokyo 103-8666 (JP)
(72) Inventor: OKANO Fumiyoshi, Kamakura-shi, Kanagawa 248-8555 (JP); SAITO Takanori, Kamakura-shi, Kanagawa 248-8555 (JP)
(74) Representative: Mewburn Ellis LLP
(86) International application number: PCT/JP2023/024125
(87) International publication number: WO 2024/005123

(57) **Abstract**

A conjugate of an antibody or a fragment thereof linked to benzodiazepine, which has immunological reactivity with a CAPRIN-1 protein having an amino acid sequence represented by any of even-numbered SEQ ID NOs among SEQ ID NOs: 2 to 30 or an amino acid sequence having 80% or more sequence identity to the amino acid sequence, is useful as an antibody-drug conjugate (ADC).

## Description

### Technical Field

The present invention relates to a conjugate of an antibody against a CAPRIN-1 protein or a fragment thereof and benzodiazepine, and medical use thereof as a therapeutic and/or preventive agent for a cancer, etc.

### Background Art

Various antibody drugs targeting specific antigenic proteins on cancer cells are applied as therapeutic drugs for cancers with fewer adverse reactions to cancer treatment because of their cancer specificity. For example, cytoplasmic-activation and proliferation-associated protein 1 (CAPRIN-1) is expressed on the cell membrane surface of many solid cancers, and antibodies against this CAPRIN-1 protein have been known to be promising in medical use for the treatment and/or prevention of cancers (Patent Literature 1).

Some benzodiazepines are known to recognize a particular DNA sequence, bind to the DNA minor groove, block DNA synthesis, and inhibit the cell growth, to thereby exert antitumor effects. Specific examples include pyrrolobenzodiazepine (PBD), indolynobenzodiazepine (IGN), and pyridinobenzodiazepine (PDD).

In recent years, studies have been made to enhance the pharmacological effects of the antibody drugs against cancers. Particularly, antibody-drug conjugates (ADCs) in which an antibody is conjugated with a drug having the strong ability to directly kill cells have been actively developed (Non Patent Literatures 1 and 2). While there are few cases of success of ADC using benzodiazepine, as an ADC comprising an anti-CD19 antibody bound to Tesirine (SG3249 derivative), which is one of the PBD derivatives, application of ZYNLONTA^{™} (loncastuximab tesirine-lpyl) to large B-cell lymphoma has been approved, and ZYNLONTA^{™} has led to a complete or partial response in 48% or more of the recurrent/intractable diffuse large B-cell lymphoma (DLBCL) cases. In addition, Rovalpituzumab Tesirine comprising Tesirine bound to an antibody against DLL-3, Vadastuximab Talirine (SGN-CD33A) comprising Talirine bound to an antibody against CD33, Camidanlumab Tesirine (Cami) comprising Tesirine bound to an antibody against CD25, SGN-CD70a comprising SGD-1882 bound to an antibody against CD70a, and the like have been developed.

### Citation List

### Patent Literature

Patent Literature 1: WO 2010/016526

### Non Patent Literature

Non Patent Literature 1: Lancet Oncol., 2016; 17: e256-62
Non Patent Literature 2: Pharm. Res., Nov. 2015; 32 (11): 3526-40

### Summary of Invention

### Technical Problem

As described above, ADC using benzodiazepine to enhance efficacy of antibody drugs against cancer has been put to practical use; however, cases of success are limited to some cancer species, and antitumor effects are also limited.

An object of the present invention is to enhance the antitumor effect of ADC using benzodiazepine compared with that of conventional techniques.

### Solution to Problem

The present inventor has conducted diligent studies and consequently completed the present invention by finding that: a conjugate of an antibody or a fragment thereof against a CAPRIN-1 protein and benzodiazepine exerts a much stronger antitumor effect than that of the antibody against the CAPRIN-1 protein or the fragment thereof used alone; and the effect of enhancing the antitumor effect by conjugating the antibody against the CAPRIN-1 protein or the fragment thereof to benzodiazepine is much superior to the effect of enhancing the antitumor effect by conjugating an existing antibody drug for a cancer to benzodiazepine.

Specifically, the present invention has the following features (1) to (15):
(1) A conjugate of an antibody or a fragment thereof linked to benzodiazepine, wherein the antibody or the fragment thereof has immunological reactivity with a CAPRIN-1 protein having an amino acid sequence represented by any of even-numbered SEQ ID NOs among SEQ ID NOs: 2 to 30 or an amino acid sequence having 80% or more sequence identity to the amino acid sequence.
(2) The conjugate according to (1), wherein the antibody or the fragment thereof has immunological reactivity with a partial polypeptide of the CAPRIN-1 protein, wherein the partial polypeptide has an amino acid sequence represented by any of SEQ ID NOs: 31 to 35 and 296 to 299, 308, and 309 or an amino acid sequence having 80% or more sequence identity to the amino acid sequence.
(3) The conjugate according to claim 1 or 2, wherein the antibody is a monoclonal antibody or a polyclonal antibody.
(4) The conjugate according to any one of (1) to (3), wherein the antibody or the fragment thereof is any of the following (A) to (M):
   (A) an antibody or a fragment thereof, which comprises a heavy chain variable region comprising complementarity-determining regions of SEQ ID NOs: 36, 37, and 38 (CDR1, CDR2, and CDR3, respectively) and a light chain variable region comprising complementarity-determining regions of SEQ ID NOs: 40, 41, and 42 (CDR1, CDR2, and CD3, respectively) and has immunological reactivity with the CAPRIN-1 protein,
   (B) an antibody or a fragment thereof, which comprises a heavy chain variable region comprising complementarity-determining regions of SEQ ID NOs: 44, 45, and 46 (CDR1, CDR2, and CDR3, respectively) and a light chain variable region comprising complementarity-determining regions of SEQ ID NOs: 48, 49, and 50 (CDR1, CDR2, and CDR3, respectively) and has immunological reactivity with the CAPRIN-1 protein,
   (C) an antibody or a fragment thereof, which comprises a heavy chain variable region comprising complementarity-determining regions of SEQ ID NOs: 52, 53, and 54 (CDR1, CDR2, and CDR3, respectively) and a light chain variable region comprising complementarity-determining regions of SEQ ID NOs: 56, 57, and 58 (CDR1, CDR2, and CDR3, respectively) and has immunological reactivity with the CAPRIN-1 protein,
   (D) an antibody or a fragment thereof, which comprises a heavy chain variable region comprising complementarity-determining regions of SEQ ID NOs: 60, 61, and 62 (CDR1, CDR2, and CDR3, respectively) and a light chain variable region comprising complementarity-determining regions of SEQ ID NOs: 64, 65, and 66 (CDR1, CDR2, and CDR3, respectively) and has immunological reactivity with the CAPRIN-1 protein,
   (E) an antibody or a fragment thereof, which comprises a heavy chain variable region comprising complementarity-determining regions of SEQ ID NOs: 170, 171, and 172 (CDR1, CDR2, and CDR3, respectively) and a light chain variable region comprising complementarity-determining regions of SEQ ID NOs: 173, 174, and 175 (CDR1, CDR2, and CDR3, respectively) and has immunological reactivity with the CAPRIN-1 protein,
   (F) an antibody or a fragment thereof, which comprises a heavy chain variable region comprising complementarity-determining regions of SEQ ID NOs: 176, 177, and 178 (CDR1, CDR2, and CDR3, respectively) and a light chain variable region comprising complementarity-determining regions of SEQ ID NOs: 179, 180, and 181 (CDR1, CDR2, and CDR3, respectively) and has immunological reactivity with the CAPRIN-1 protein,
   (G) an antibody or a fragment thereof, which comprises a heavy chain variable region comprising complementarity-determining regions of SEQ ID NOs: 182, 183, and 184 (CDR1, CDR2, and CDR3, respectively) and a light chain variable region comprising complementarity-determining regions of SEQ ID NOs: 185, 186, and 187 (CDR1, CDR2, and CDR3, respectively) and has immunological reactivity with the CAPRIN-1 protein,
   (H) an antibody or a fragment thereof, which comprises a heavy chain variable region comprising complementarity-determining regions of SEQ ID NOs: 188, 189, and 190 (CDR1, CDR2, and CDR3, respectively) and a light chain variable region comprising complementarity-determining regions of SEQ ID NOs: 191, 192, and 193 (CDR1, CDR2, and CDR3, respectively) and has immunological reactivity with the CAPRIN-1 protein,
   (I) an antibody or a fragment thereof, which comprises a heavy chain variable region comprising complementarity-determining regions of SEQ ID NOs: 146, 147, and 148 (CDR1, CDR2, and CDR3, respectively) and a light chain variable region comprising complementarity-determining regions of SEQ ID NOs: 149, 150, and 151 (CDR1, CDR2, and CDR3, respectively) and has immunological reactivity with the CAPRIN-1 protein,
   (J) an antibody or a fragment thereof, which comprises a heavy chain variable region comprising complementarity-determining regions of SEQ ID NOs: 272, 273, and 274 (CDR1, CDR2, and CDR3, respectively) and a light chain variable region comprising complementarity-determining regions of SEQ ID NOs: 275, 276, and 277 (CDR1, CDR2, and CDR3, respectively) and has immunological reactivity with the CAPRIN-1 protein,
   (K) an antibody or a fragment thereof, which comprises a heavy chain variable region comprising complementarity-determining regions of SEQ ID NOs: 290, 291, and 292 (CDR1, CDR2, and CDR3, respectively) and a light chain variable region comprising complementarity-determining regions of SEQ ID NOs: 293, 294, and 295 (CDR1, CDR2, and CDR3, respectively) and has immunological reactivity with the CAPRIN-1 protein,
   (L) an antibody or a fragment thereof, which comprises a heavy chain variable region comprising complementarity-determining regions of SEQ ID NOs: 301, 302, and 303 (CDR1, CDR2, and CDR3, respectively) and a light chain variable region comprising complementarity-determining regions of SEQ ID NOs: 305, 306, and 307 (CDR1, CDR2, and CDR3, respectively) and has immunological reactivity with the CAPRIN-1 protein, and
   (M) an antibody or a fragment thereof, which comprises a heavy chain variable region comprising complementarity-determining regions of SEQ ID NOs: 134, 135, and 136 (CDR1, CDR2, and CDR3, respectively) and a light chain variable region comprising complementarity-determining regions of SEQ ID NOs: 137, 138, and 139 (CDR1, CDR2, and CDR3, respectively) and has immunological reactivity with the CAPRIN-1 protein.
(5) The conjugate according to any one of (1) to (4), wherein the antibody or the fragment thereof is any of the following (a) to (al):
   (a) an antibody or a fragment thereof, comprising a heavy chain variable region comprising the amino acid sequence of SEQ ID NO: 39 and a light chain variable region comprising the amino acid sequence of SEQ ID NO: 43,
   (b) an antibody or a fragment thereof, comprising a heavy chain variable region comprising the amino acid sequence of SEQ ID NO: 47 and a light chain variable region comprising the amino acid sequence of SEQ ID NO: 51,
   (c) an antibody or a fragment thereof, comprising a heavy chain variable region comprising the amino acid sequence of SEQ ID NO: 55 and a light chain variable region comprising the amino acid sequence of SEQ ID NO: 59,
   (d) an antibody or a fragment thereof, comprising a heavy chain variable region comprising the amino acid sequence of SEQ ID NO: 63 and a light chain variable region comprising the amino acid sequence of SEQ ID NO: 67,
   (e) an antibody or a fragment thereof, comprising a heavy chain variable region comprising the amino acid sequence of SEQ ID NO: 68 and a light chain variable region comprising the amino acid sequence of SEQ ID NO: 69,
   (f) an antibody or a fragment thereof, comprising a heavy chain variable region comprising the amino acid sequence of SEQ ID NO: 70 and a light chain variable region comprising the amino acid sequence of SEQ ID NO: 71,
   (g) an antibody or a fragment thereof, comprising a heavy chain variable region comprising the amino acid sequence of SEQ ID NO: 72 and a light chain variable region comprising the amino acid sequence of SEQ ID NO: 73,
   (h) an antibody or a fragment thereof, comprising a heavy chain variable region comprising the amino acid sequence of SEQ ID NO: 74 and a light chain variable region comprising the amino acid sequence of SEQ ID NO: 75,
   (i) an antibody or a fragment thereof, comprising a heavy chain variable region comprising the amino acid sequence of SEQ ID NO: 76 and a light chain variable region comprising the amino acid sequence of SEQ ID NO: 77,
   (j) an antibody or a fragment thereof, comprising a heavy chain variable region comprising the amino acid sequence of SEQ ID NO: 78 and a light chain variable region comprising the amino acid sequence of SEQ ID NO: 79,
   (k) an antibody or a fragment thereof, comprising a heavy chain variable region comprising the amino acid sequence of SEQ ID NO: 80 and a light chain variable region comprising the amino acid sequence of SEQ ID NO: 81,
   (l) an antibody or a fragment thereof, comprising a heavy chain variable region comprising the amino acid sequence of SEQ ID NO: 82 and a light chain variable region comprising the amino acid sequence of SEQ ID NO: 83,
   (m) an antibody or a fragment thereof, comprising a heavy chain variable region comprising the amino acid sequence of SEQ ID NO: 84 and a light chain variable region comprising the amino acid sequence of SEQ ID NO: 85,
   (n) an antibody or a fragment thereof, comprising a heavy chain variable region comprising the amino acid sequence of SEQ ID NO: 86 and a light chain variable region comprising the amino acid sequence of SEQ ID NO: 87,
   (o) an antibody or a fragment thereof, comprising a heavy chain variable region comprising the amino acid sequence of SEQ ID NO: 88 and a light chain variable region comprising the amino acid sequence of SEQ ID NO: 89,
   (p) an antibody or a fragment thereof, comprising a heavy chain variable region comprising the amino acid sequence of SEQ ID NO: 90 and a light chain variable region comprising the amino acid sequence of SEQ ID NO: 91,
   (q) an antibody or a fragment thereof, comprising a heavy chain variable region comprising the amino acid sequence of SEQ ID NO: 92 and a light chain variable region comprising the amino acid sequence of SEQ ID NO: 93,
   (r) an antibody or a fragment thereof, comprising a heavy chain variable region comprising the amino acid sequence of SEQ ID NO: 94 and a light chain variable region comprising the amino acid sequence of SEQ ID NO: 95,
   (s) an antibody or a fragment thereof, comprising a heavy chain variable region comprising the amino acid sequence of SEQ ID NO: 96 and a light chain variable region comprising the amino acid sequence of SEQ ID NO: 97,
   (t) an antibody or a fragment thereof, comprising a heavy chain variable region comprising the amino acid sequence of SEQ ID NO: 98 and a light chain variable region comprising the amino acid sequence of SEQ ID NO: 99,
   (u) an antibody or a fragment thereof, comprising a heavy chain variable region comprising the amino acid sequence of SEQ ID NO: 100 and a light chain variable region comprising the amino acid sequence of SEQ ID NO: 101,
   (v) an antibody or a fragment thereof, comprising a heavy chain variable region comprising the amino acid sequence of SEQ ID NO: 102 and a light chain variable region comprising the amino acid sequence of SEQ ID NO: 103,
   (w) an antibody or a fragment thereof, comprising a heavy chain variable region comprising the amino acid sequence of SEQ ID NO: 104 and a light chain variable region comprising the amino acid sequence of SEQ ID NO: 105,
   (x) an antibody or a fragment thereof, comprising a heavy chain variable region comprising the amino acid sequence of SEQ ID NO: 106 and a light chain variable region comprising the amino acid sequence of SEQ ID NO: 107,
   (y) an antibody or a fragment thereof, comprising a heavy chain variable region comprising the amino acid sequence of SEQ ID NO: 108 and a light chain variable region comprising the amino acid sequence of SEQ ID NO: 109,
   (z) an antibody or a fragment thereof, comprising a heavy chain variable region comprising the amino acid sequence of SEQ ID NO: 110 and a light chain variable region comprising the amino acid sequence of SEQ ID NO: 111,
   (aa) an antibody or a fragment thereof, comprising a heavy chain variable region comprising the amino acid sequence of SEQ ID NO: 112 and a light chain variable region comprising the amino acid sequence of SEQ ID NO: 113,
   (ab) an antibody or a fragment thereof, comprising a heavy chain variable region comprising the amino acid sequence of SEQ ID NO: 114 and a light chain variable region comprising the amino acid sequence of SEQ ID NO: 115,
   (ac) an antibody or a fragment thereof, comprising a heavy chain variable region comprising the amino acid sequence of SEQ ID NO: 116 and a light chain variable region comprising the amino acid sequence of SEQ ID NO: 117,
   (ad) an antibody or a fragment thereof, comprising a heavy chain variable region comprising the amino acid sequence of SEQ ID NO: 118 and a light chain variable region comprising the amino acid sequence of SEQ ID NO: 119,
   (ae) an antibody or a fragment thereof, comprising a heavy chain variable region comprising the amino acid sequence of SEQ ID NO: 120 and a light chain variable region comprising the amino acid sequence of SEQ ID NO: 121,
   (af) an antibody or a fragment thereof, comprising a heavy chain variable region comprising the amino acid sequence of SEQ ID NO: 122 and a light chain variable region comprising the amino acid sequence of SEQ ID NO: 123,
   (ag) an antibody or a fragment thereof, comprising a heavy chain variable region comprising the amino acid sequence of SEQ ID NO: 124 and a light chain variable region comprising the amino acid sequence of SEQ ID NO: 125,
   (ah) an antibody or a fragment thereof, comprising a heavy chain variable region comprising the amino acid sequence of SEQ ID NO: 126 and a light chain variable region comprising the amino acid sequence of SEQ ID NO: 127,
   (ai) an antibody or a fragment thereof, comprising a heavy chain variable region comprising the amino acid sequence of SEQ ID NO: 128 and a light chain variable region comprising the amino acid sequence of SEQ ID NO: 129,
   (aj) an antibody or a fragment thereof, comprising a heavy chain variable region comprising the amino acid sequence of SEQ ID NO: 130 and a light chain variable region comprising the amino acid sequence of SEQ ID NO: 131,
   (ak) an antibody or a fragment thereof, comprising a heavy chain variable region comprising the amino acid sequence of SEQ ID NO: 132 and a light chain variable region comprising the amino acid sequence of SEQ ID NO: 133, and
   (al) an antibody or a fragment thereof, comprising a heavy chain variable region comprising the amino acid sequence of SEQ ID NO: 300 and a light chain variable region comprising the amino acid sequence of SEQ ID NO: 304.
(6) The conjugate according to any one of (1) to (5), wherein the antibody is a human antibody, a humanized antibody, a chimeric antibody, or a single-chain antibody.
(7) The conjugate according to any one of (1) to (6), wherein the antibody or the fragment thereof is linked to benzodiazepine via a linker.
(8) The conjugate according to any one of (1) to (7), wherein the benzodiazepine is benzodiazepine having antitumor activity.
(9) The conjugate according to any one of (1) to (8), wherein the benzodiazepine having antitumor activity is pyrrolobenzodiazepine (PBD), indolynobenzodiazepine (IGN), pyridinobenzodiazepine (PDD), or isoquinolidinobenzodiazepine (IQB).
(10) The conjugate according to any one of (1) to (9), wherein the benzodiazepine is DSB-120, SJG-136 (SG2000), DC-81, DSB-120, SJG-136, SG2057, SG2202, SG2285, SGD-1882, SGD-1910, SG3199, SG3249, SG2219, IMGN779, IMGN632, (S)-N-(4-aminophenyl)-4-(4-(4-(4-((2-methoxy-12-oxo-6a,7,8,9,10,12-hexahydrobenzo[e]pyrido[1,2-a][1,4]diazepin-3-yl)oxy)butanamide)-1-methyl-1H-pyrrole-2-carboxamide)phenyl)-1-methyl-1H-pyrrole-2-carboxamide, D211, D221, D231, GWL-78, KMR-28-39, or a derivative of any thereof.
(11) The conjugate according to (10), wherein the derivative is Tesirine (SG3249 derivative), Talirine (SGD-1910 derivative), SG3364, SG3227, SG3140 (MC-Phe-Lys-PAB-SG2057), SG3170, SG3203 (MC-Phe-Lys-PAB-SG2057), SG3231, SG3400, SG3376, DGN642, DGN549, FGX5-67, FGX-2-62, or FGX11-38.
(12) A pharmaceutical composition for the treatment and/or prevention of a cancer, comprising the conjugate according to any one of (1) to (11) as an active ingredient.
(13) The pharmaceutical composition according to (12), wherein the cancer is a cancer expressing a CAPRIN-1 protein on the cell membrane surface.
(14) The pharmaceutical composition according to (12) or (13), wherein the cancer is breast cancer, kidney cancer, pancreatic cancer, colorectal cancer, lung cancer, brain tumor, stomach cancer, uterine cancer, ovary cancer, prostate cancer, bladder cancer, esophagus cancer, leukemia, lymphoma, liver cancer, gallbladder cancer, bile duct cancer, sarcoma, mastocytoma, melanoma, adrenal cortex cancer, Ewing's tumor, Hodgkin's lymphoma, mesothelioma, multiple myeloma, testicle cancer, thyroid cancer, head and neck cancer, or urothelial carcinoma.
(15) A method for treating and/or preventing a cancer, comprising administering the conjugate according to any one of (1) to (11) or the pharmaceutical composition according to any one of (12) to (14) to a subject.

### Advantageous Effects of Invention

The conjugate according to the present invention not only exerts a much stronger antitumor effect than that of an antibody against a CAPRIN-1 protein or a fragment thereof used alone but is superior in antitumor effect to a known conjugate of an antibody drug for a cancer and benzodiazepine. Also, the effect of enhancing the antitumor effect achieved by conjugating an antibody against a CAPRIN-1 protein or a fragment thereof to benzodiazepine is superior to the effect of enhancing the antitumor effect by conjugating an existing antibody drug for a cancer to benzodiazepine. Thus, the conjugate according to the present invention is effective for the treatment or prevention of a cancer.

### Description of Embodiments

The conjugate of an antibody or a fragment thereof against a CAPRIN-1 protein (hereinafter, referred to as an "anti-CAPRIN-1 antibody") and benzodiazepine used in the present invention can be evaluated for its antitumor activity, as mentioned later, by examining *in vivo* the inhibition of tumor growth in a cancer-bearing animal.

In the present invention, the "conjugate" refers to an antibody linked to benzodiazepine via a covalent bond. The linking between the antibody and benzodiazepine may be done by a linker.

The anti-CAPRIN-1 antibody that is a constituent of the conjugate according to the present invention may be a monoclonal antibody or a polyclonal antibody and is preferably a monoclonal antibody. The anti-CAPRIN-1 antibody may be any type of antibody as long as the conjugate of the present invention can exert antitumor activity. The antibody may be a recombinant antibody, a human antibody, a humanized antibody, a chimeric antibody, or a non-human animal antibody.

Benzodiazepine that is a constituent of the conjugate of the present invention is known as a substance that exerts toxicity on cancer cells by recognizing a particular DNA sequence to bind to the DNA minor groove and blocking DNA synthesis to inhibit the cell growth.

The subject to be treated and/or prevented for cancer according to the present invention is a mammal such as a human, a pet animal, livestock, or a sport animal, and a preferred subject is a human.

Hereinafter, the anti-CAPRIN-1 antibody, benzodiazepine, the conjugate of the anti-CAPRIN-1 antibody and benzodiazepine, the pharmaceutical composition comprising the conjugate, and the method for treating and/or preventing a cancer using the conjugate, according to the present invention will be described.

### <Anti-CAPRIN-1 antibody>

Among CAPRIN-1 proteins having an amino acid sequence represented by any of even-numbered SEQ ID NOs among SEQ ID NOs: 2 to 30 and having immunological reactivity with the anti-CAPRIN-1 antibody used in the present invention, the amino acid sequences represented by SEQ ID NOs: 6, 8, 10, 12, and 14 are the amino acid sequences of canine CAPRIN-1 proteins; the amino acid sequences represented by SEQ ID NOs: 2 and 4 are the amino acid sequences of human CAPRIN-1 proteins; the amino acid sequence represented by SEQ ID NO: 16 is the amino acid sequence of a bovine CAPRIN-1 protein; the amino acid sequence represented by SEQ ID NO: 18 is the amino acid sequence of an equine CAPRIN-1 protein; the amino acid sequences represented by SEQ ID NOs: 20 to 28 are the amino acid sequences of mouse CAPRIN-1 proteins; and the amino acid sequence represented by SEQ ID NO: 30 is the amino acid sequence of a chicken CAPRIN-1 protein.

The anti-CAPRIN-1 antibody used in the present invention may have immunological reactivity with a variant of the CAPRIN-1 protein having 80% or more, preferably 90% or more, more preferably 95% or more, further preferably 99% or more sequence identity to the amino acid sequence represented by any of even-numbered SEQ ID NOs among SEQ ID NOs: 2 to 30. In this context, the term "% sequence identity" means a percentage (%) of the number of identical amino acids (or bases) to the total number of amino acids (or bases) when two sequences are aligned so that the maximum degree of similarity can be achieved with or without introducing a gap.

In the present invention, the anti-CAPRIN-1 antibody that is used for preparing the conjugate means an antibody or an antigen-binding fragment thereof having immunological reactivity with a full-length CAPRIN-1 protein or a fragment thereof as an antigen. In this context, the "immunological reactivity" means the property of the antibody binding to the CAPRIN-1 protein or a partial polypeptide thereof *in vivo.*

The anti-CAPRIN-1 antibody used in the present invention may be a monoclonal antibody or a polyclonal antibody.

The polyclonal antibody having immunological reactivity with the full-length CAPRIN-1 protein or the fragment thereof (anti-CAPRIN-1 polyclonal antibody) can be obtained, for example, by immunizing a mouse, a human antibody-producing mouse, a rat, a rabbit, a chicken, or the like with a naturally occurring CAPRIN-1 protein, or a fusion protein thereof with GST or the like, or a partial peptide thereof and obtaining serum therefrom, and applying the obtained serum to ammonium sulfate precipitation, protein A, protein G, a DEAE ion-exchange column, an affinity column linked to a CAPRIN-1 protein or a partial peptide, or the like.

As for the full-length CAPRIN-1 protein or the fragment thereof to be used in the immunization, the nucleotide sequences and amino acid sequences of CAPRIN-1 and homologs thereof are available, for example, by accessing GenBank (NCBI, U.S.A.) and using an algorithm such as BLAST or FASTA (Karlin and Altschul, Proc. Natl. Acad. Sci., U.S.A., 90: 5873-5877, 1993; and Altschul et al., Nucleic Acids Res., 25: 3389-3402, 1997). Also, a method for preparing the CAPRIN-1 protein is available with reference to WO 2014/012479, or can be carried out using, for example, cells expressing the CAPRIN-1 protein.

The monoclonal antibody having immunological reactivity with the full-length CAPRIN-1 protein or the fragment thereof (anti-CAPRIN-1 monoclonal antibody) can be obtained, for example, by: administering SK-BR-3 (breast cancer cells expressing CAPRIN-1) or the full-length CAPRIN-1 protein or the fragment thereof, or the like to a mouse for immunization; fusing spleen cells separated from the mouse with myeloma cells; and selecting a clone producing anti-CAPRIN-1 monoclonal antibodies from among the obtained fusion cells (hybridomas). The antibody produced from the hybridoma thus selected can be prepared in the same way as the method for purifying the polyclonal antibody mentioned above.

The antibody used in the present invention includes human antibodies, humanized antibodies, chimeric antibodies, and non-human animal antibodies.

The human antibody can be obtained by: sensitizing EB virus-infected human lymphocytes with the protein, protein-expressing cells, or lysates thereof; fusing the sensitized lymphocytes with human-derived myeloma cells such as U266 cells; and obtaining an antibody having immunological reactivity with the full-length CAPRIN-1 protein or the fragment thereof from the obtained fusion cells.

The humanized antibody, also called a reshaped human antibody, is an engineered antibody. The humanized antibody is constructed by grafting complementarity-determining regions of an antibody derived from an immunized animal onto complementarity-determining regions of a human antibody. A genetic engineering technique commonly used for constructing humanized antibodies is also well-known. Specifically, DNA sequences designed to link complementarity-determining regions of, for example, a mouse or rabbit antibody, to framework regions of a human antibody are synthesized by PCR from several prepared oligonucleotides having overlapping terminal portions. The obtained DNAs are ligated with DNAs encoding human antibody constant regions. The resulting ligation products are incorporated into expression vectors, which are then transferred to hosts for antibody production to obtain the antibody of interest (see European Patent Application Publication No. EP239400 and International Publication No. WO 96/02576). The framework regions of a human antibody connected via the complementarity-determining regions are selected so that the complementarity-determining regions form a favorable antigen-binding site. If necessary, an amino acid in the framework regions of antibody variable regions may be substituted so that the complementarity-determining regions of a reshaped human antibody form an appropriate antigen-binding site (Sato K. et al., Cancer Research 1993, 53: 851-856). In addition, these framework regions may be replaced with framework regions derived from various human antibodies (see WO 99/51743).

Antibodies are typically heteromultimeric glycoproteins each comprising at least two heavy chains and two light chains. The antibodies are each composed of two identical light chains and two identical heavy chains. Each heavy chain has a heavy chain variable region at one end, followed by a series of constant regions. Each light chain has a light chain variable region at one end, followed by a series of constant regions. The variable regions contain certain variable regions called complementarity-determining regions (CDRs) and impart binding specificity to the antibody. Portions relatively conserved in the variable regions are called framework regions (FRs). The complete heavy chain and light chain variable regions each contain four FRs connected via three CDRs (CDR1 to CDR3).

The sequences of human-derived heavy chain and light chain constant regions and variable regions are available from, for example, NCBI (U.S.A.; GenBank, UniGene, etc.). For example, the following sequences can be referred to: Accession No. J00228 for a human IgG1 heavy chain constant region; Accession No. J00230 for a human IgG2 heavy chain constant region; Accession Nos. V00557, X64135, X64133, etc., for a human light chain κ constant region; and Accession Nos. X64132, X64134, etc., for a human light chain λ constant region.

A chimeric antibody is an antibody prepared from a combination of sequences derived from different animals and may be, for example, an antibody composed of heavy chain and light chain variable regions of a mouse antibody and constant regions of heavy chain and light chain variable regions of a human antibody. The chimeric antibody can be prepared using a method known in the art and is obtained, for example, by: ligating DNAs encoding the antibody V regions to DNAs encoding the human antibody C regions; incorporating the resulting ligation products into expression vectors; and transferring the vectors into hosts for antibody production.

The non-human animal antibody is obtained by immunizing an animal with a sensitizing antigen according to a method known in the art and, as a general method, by intraperitoneally, intracutaneously, or subcutaneously injecting a sensitizing antigen to an animal such as a mouse. For the injection of the sensitizing antigen, the antigen is mixed in an appropriate amount with various adjuvants including CFA (complete Freund's adjuvant), and the mixture is administered to the animal a plurality of times. The animal is immunized and then verified to contain anti-CAPRIN-1 antibodies in serum. The serum can be obtained and applied, as mentioned above, to ammonium sulfate precipitation, protein A, protein G, a DEAE ion-exchange column, an affinity column bound with a CAPRIN-1 protein or a partial peptide, or the like to obtain the non-human animal antibody. In the case of obtaining a monoclonal antibody from a non-human animal, immunocytes can be collected from an immunized animal and subjected to cell fusion with myeloma cells to obtain the monoclonal antibody. The cell fusion between the immunocytes and the myeloma cells can be carried out according to a method known in the art (see Kohler, G. and Milstein, C. Methods Enzymol., 1981, 73, 3-46).

The antibody used in the present invention may be also obtained as a recombinant antibody produced using a genetic engineering technique by cloning genes of the antibody from a hybridoma, incorporating the antibody genes into appropriate vectors, and transferring the vectors into hosts (see Carl, A.K. Borrebaeck, James, W. Larrick, THERAPEUTIC MONOCLONAL ANTIBODIES, Published in the United Kingdom by MACMILLAN PUBLISHERS LTD, 1990).

The anti-CAPRIN-1 antibody that is used for obtaining the conjugate of the present invention may be an antibody in which an amino acid in a variable region (e.g., FR) or a constant region is substituted with another amino acid. The amino acid substitution is the substitution of one or more, for example, less than 15, less than 10, 8 or less, 6 or less, 5 or less, 4 or less, 3 or less, or 2 or less amino acids, preferably 1 to 9 amino acids. The substituted antibody should be an antibody that has the property of specifically binding to the antigen and binding affinity for the antigen equivalent to or greater than those of the corresponding unsubstituted antibody and causes no rejection when applied to humans.

The anti-CAPRIN-1 antibody used in the present invention is expected to have a stronger antitumor effect, the higher binding affinity for the CAPRIN-1 protein on cancer cell surface the antibody has. Its association constant (affinity constant) Ka (kon/koff) is preferably at least 10⁷ M⁻¹, at least 10⁸ M⁻¹, at least 5 x 10⁸ M⁻¹, at least 10⁹ M⁻¹, at least 5 x 10⁹ M⁻¹, at least 10¹⁰ M⁻¹, at least 5 x 10¹⁰ M⁻¹, at least 10¹¹ M⁻¹, at least 5 x 10¹¹ M⁻¹, at least 10¹² M⁻¹, or at least 10¹³ M⁻¹.

The binding activity of the anti-CAPRIN-1 antibody used in the present invention against effector cells can be improved by substituting one, two or several amino acids in the heavy chain constant region of the antibody or by removing fucose added to N-acetylglucosamine in a N-glycoside-linked sugar chain attached to the heavy chain constant region. Such an antibody may have the amino acid substitution alone or may be in the form of a composition comprising a fucosylated antibody.

The antibody in which one, two or several amino acids in the heavy chain constant region are substituted can be prepared with reference to, for example, WO 2004/063351, WO 2011/120135, U.S. Patent No. 8388955, WO 2011/005481, U.S. Patent No. 6737056, and/or WO 2005/063351.

The antibody lacking fucose added to N-acetylglucosamine in a N-glycoside-linked sugar chain in the heavy chain constant region, or cells producing the antibody, can be prepared with reference to U.S. Patent No. 6602684, European Patent No. 1914244, and/or U.S. Patent No. 7579170. A composition of the antibody lacking fucose added to N-acetylglucosamine in a N-glycoside-linked sugar chain attached to the heavy chain constant region, and the antibody having the fucose, or cells producing the composition can be prepared with reference to, for example, U.S. Patent No. 8642292.

Methods for preparing and purifying the anti-CAPRIN-1 polyclonal antibody, the anti-CAPRIN-1 monoclonal antibody, and the antibody used in the present invention, and a method for preparing the CAPRIN-1 protein or the partial polypeptide thereof to be used in immunization can be carried out with reference to WO 2010/016526, WO 2011/096517, WO 2011/096528, WO 2011/096519, WO 2011/096533, WO 2011/096534, WO 2011/096535, WO 2013/018886, WO 2013/018894, WO 2013/018892, WO 2013/018891, WO 2013/018889, WO 2013/018883, WO 2013/125636, WO 2013/125654, WO 2013/125630, WO 2013/125640, WO 2013/147169, WO 2013/147176 and WO 2015/020212.

Specific examples of the anti-CAPRIN-1 antibody according to the present invention include anti-CAPRIN-1 antibodies disclosed in WO 2010/016526, WO 2011/096517, WO 2011/096528, WO 2011/096519, WO 2011/096533, WO 2011/096534, WO 2011/096535, WO 2013/018886, WO 2013/018894, WO 2013/018892, WO 2013/018891, WO 2013/018889, WO 2013/018883, WO 2013/125636, WO 2013/125654, WO 2013/125630, WO 2013/125640, WO 2013/147169, WO 2013/147176 and WO 2015/020212 mentioned above. Preferred examples of the anti-CAPRIN-1 antibody include the following.

An antibody or a fragment thereof having immunological reactivity with the amino acid sequence represented by SEQ ID NO: 2 or SEQ ID NO: 4 or a partial polypeptide of the CAPRIN-1 protein having an amino acid sequence having 80% or more (preferably 85% or more, more preferably 90% or more, further preferably 95% or more, still further preferably 99% or more) sequence identity to the amino acid sequence represented by SEQ ID NO: 2 or SEQ ID NO: 4.

An antibody or a fragment thereof having immunological reactivity with a partial polypeptide of the CAPRIN-1 protein having the amino acid sequence represented by SEQ ID NO: 31 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 36, 37, and 38 (CDR1, CDR2, and CDR3, respectively) and a light chain variable region comprising complementarity-determining regions of SEQ ID NOs: 40, 41, and 42 (CDR1, CDR2, and CDR3, respectively) and has immunological reactivity with the CAPRIN-1 protein; an antibody or a fragment thereof which comprises a heavy chain variable region comprising complementarity-determining regions of SEQ ID NOs: 140, 141, and 142 (CDR1, CDR2, and CDR3, respectively) and a light chain variable region comprising complementarity-determining regions of SEQ ID NOs: 143, 144, and 145 (CDR1, CDR2, and CDR3, respectively) and has immunological reactivity with the CAPRIN-1 protein; or an antibody or a fragment thereof which comprises a heavy chain variable region comprising complementarity-determining regions of SEQ ID NOs: 164, 165, and 166 (CDR1, CDR2, and CDR3, respectively) and a light chain variable region comprising complementarity-determining regions of SEQ ID NOs: 167, 168, and 169 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 39 and a light chain variable region comprising the amino acid sequence of SEQ ID NO: 43; an antibody or a fragment thereof comprising a heavy chain variable region comprising the amino acid sequence of SEQ ID NO: 70 and a light chain variable region comprising the amino acid sequence of SEQ ID NO: 71; or an antibody or a fragment thereof comprising a heavy chain variable region comprising the amino acid sequence of SEQ ID NO: 78 and a light chain variable region comprising the amino acid sequence of SEQ ID NO: 79.

An antibody or a fragment thereof having immunological reactivity with a partial polypeptide of the CAPRIN-1 protein having the amino acid sequence represented by SEQ ID NO: 33 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 60, 61, and 62 (CDR1, CDR2, and CDR3, respectively) and a light chain variable region comprising complementarity-determining regions of SEQ ID NOs: 64, 65, and 66 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 63 and a light chain variable region comprising the amino acid sequence of SEQ ID NO: 67.

An antibody or a fragment thereof having immunological reactivity with a partial polypeptide of the CAPRIN-1 protein having the amino acid sequence represented by SEQ ID NO: 32 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 52, 53, and 54 (CDR1, CDR2, and CDR3, respectively) and a light chain variable region comprising complementarity-determining regions of SEQ ID NOs: 56, 57, and 58 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 55 and a light chain variable region comprising the amino acid sequence of SEQ ID NO: 59.

An antibody or a fragment thereof having immunological reactivity with a partial polypeptide of the CAPRIN-1 protein having the amino acid sequence represented by SEQ ID NO: 34 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 170, 171, and 172 (CDR1, CDR2, and CDR3, respectively) and a light chain variable region comprising complementarity-determining regions of SEQ ID NOs: 173, 174, and 175 (CDR1, CDR2, and CDR3, respectively) and has immunological reactivity with the CAPRIN-1 protein; or an antibody or a fragment thereof which comprises a heavy chain variable region comprising complementarity-determining regions of SEQ ID NOs: 176, 177, and 178 (CDR1, CDR2, and CDR3, respectively) and a light chain variable region comprising complementarity-determining regions of SEQ ID NOs: 179, 180, and 181 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 80 and a light chain variable region comprising the amino acid sequence of SEQ ID NO: 81; or an antibody or a fragment thereof comprising a heavy chain variable region comprising the amino acid sequence of SEQ ID NO: 82 and a light chain variable region comprising the amino acid sequence of SEQ ID NO: 83.

An antibody or a fragment thereof having immunological reactivity with a partial polypeptide of the CAPRIN-1 protein having the amino acid sequence represented by SEQ ID NO: 35 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 182, 183, and 184 (CDR1, CDR2, and CDR3, respectively) and a light chain variable region comprising complementarity-determining regions of SEQ ID NOs: 185, 186, and 187 (CDR1, CDR2, and CDR3, respectively) and has immunological reactivity with the CAPRIN-1 protein; or an antibody or a fragment thereof which comprises a heavy chain variable region comprising complementarity-determining regions of SEQ ID NOs: 188, 189, and 190 (CDR1, CDR2, and CDR3, respectively) and a light chain variable region comprising complementarity-determining regions of SEQ ID NOs: 191, 192, and 193 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 84 and a light chain variable region comprising the amino acid sequence of SEQ ID NO: 85; or an antibody or a fragment thereof comprising a heavy chain variable region comprising the amino acid sequence of SEQ ID NO: 86 and a light chain variable region comprising the amino acid sequence of SEQ ID NO: 87.

An antibody or a fragment thereof which comprises a heavy chain variable region comprising complementarity-determining regions of SEQ ID NOs: 44, 45, and 46 (CDR1, CDR2, and CDR3, respectively) and a light chain variable region comprising complementarity-determining regions of SEQ ID NOs: 48, 49, and 50 (CDR1, CDR2, and CDR3, respectively) and has immunological reactivity with the CAPRIN-1 protein. Preferably, an antibody or a fragment thereof comprising a heavy chain variable region comprising the amino acid sequence of SEQ ID NO: 47 and a light chain variable region comprising the amino acid sequence of SEQ ID NO: 51.

An antibody or a fragment thereof having immunological reactivity with a partial polypeptide of the CAPRIN-1 protein having the amino acid sequence represented by SEQ ID NO: 296 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 146, 147, and 148 (CDR1, CDR2, and CDR3, respectively) and a light chain variable region comprising complementarity-determining regions of SEQ ID NOs: 149, 150, and 151 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 72 and a light chain variable region comprising the amino acid sequence of SEQ ID NO: 73.

An antibody or a fragment thereof having immunological reactivity with a partial polypeptide of the CAPRIN-1 protein having the amino acid sequence represented by SEQ ID NO: 297 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 272, 273, and 274 (CDR1, CDR2, and CDR3, respectively) and a light chain variable region comprising complementarity-determining regions of SEQ ID NOs: 275, 276, and 277 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 114 and a light chain variable region comprising the amino acid sequence of SEQ ID NO: 115.

An antibody or a fragment thereof having immunological reactivity with a partial polypeptide of the CAPRIN-1 protein having the amino acid sequence represented by SEQ ID NO: 298 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 290, 291, and 292 (CDR1, CDR2, and CDR3, respectively) and a light chain variable region comprising complementarity-determining regions of SEQ ID NOs: 293, 294, and 295 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 120 and a light chain variable region comprising the amino acid sequence of SEQ ID NO: 121.

An antibody or a fragment thereof having immunological reactivity with a partial polypeptide of the CAPRIN-1 protein having the amino acid sequence represented by SEQ ID NO: 299 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 301, 302, and 303 (CDR1, CDR2, and CDR3, respectively) and a light chain variable region comprising complementarity-determining regions of SEQ ID NOs: 305, 306, and 307 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 300 and a light chain variable region comprising the amino acid sequence of SEQ ID NO: 304.

An antibody or a fragment thereof having immunological reactivity with a partial polypeptide of the CAPRIN-1 protein having the amino acid sequence represented by SEQ ID NO: 308 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 134, 135, and 136 (CDR1, CDR2, and CDR3, respectively) and a light chain variable region comprising complementarity-determining regions of SEQ ID NOs: 137, 138, and 139 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 68 and a light chain variable region comprising the amino acid sequence of SEQ ID NO: 69.

An antibody or a fragment thereof having immunological reactivity with a partial polypeptide of the CAPRIN-1 protein having the amino acid sequence represented by SEQ ID NO: 309 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 134, 135, and 136 (CDR1, CDR2, and CDR3, respectively) and a light chain variable region comprising complementarity-determining regions of SEQ ID NOs: 137, 138, and 139 (CDR1, CDR2, and CDR3, respectively) and has immunological reactivity with the CAPRIN-1 protein. More preferably, an antibody or a fragment thereof comprising a heavy chain variable region comprising the amino acid sequence of SEQ ID NO: 68 and a light chain variable region comprising the amino acid sequence of SEQ ID NO: 69.

Also, the following anti-CAPRIN-1 antibodies are preferably used.

An antibody or a fragment thereof comprising a heavy chain variable region comprising the amino acid sequence of SEQ ID NO: 68 and a light chain variable region comprising the amino acid sequence of SEQ ID NO: 69.

An antibody or a fragment thereof comprising a heavy chain variable region comprising the amino acid sequence of SEQ ID NO: 70 and a light chain variable region comprising the amino acid sequence of SEQ ID NO: 71.

An antibody or a fragment thereof comprising a heavy chain variable region comprising the amino acid sequence of SEQ ID NO: 72 and a light chain variable region comprising the amino acid sequence of SEQ ID NO: 73.

An antibody or a fragment thereof comprising a heavy chain variable region comprising the amino acid sequence of SEQ ID NO: 74 and a light chain variable region comprising the amino acid sequence of SEQ ID NO: 75.

An antibody or a fragment thereof comprising a heavy chain variable region comprising the amino acid sequence of SEQ ID NO: 76 and a light chain variable region comprising the amino acid sequence of SEQ ID NO: 77.

An antibody or a fragment thereof comprising a heavy chain variable region comprising the amino acid sequence of SEQ ID NO: 78 and a light chain variable region comprising the amino acid sequence of SEQ ID NO: 79.

An antibody or a fragment thereof comprising a heavy chain variable region comprising the amino acid sequence of SEQ ID NO: 80 and a light chain variable region comprising the amino acid sequence of SEQ ID NO: 81.

An antibody or a fragment thereof comprising a heavy chain variable region comprising the amino acid sequence of SEQ ID NO: 82 and a light chain variable region comprising the amino acid sequence of SEQ ID NO: 83.

An antibody or a fragment thereof comprising a heavy chain variable region comprising the amino acid sequence of SEQ ID NO: 84 and a light chain variable region comprising the amino acid sequence of SEQ ID NO: 85.

An antibody or a fragment thereof comprising a heavy chain variable region comprising the amino acid sequence of SEQ ID NO: 86 and a light chain variable region comprising the amino acid sequence of SEQ ID NO: 87.

An antibody or a fragment thereof comprising a heavy chain variable region comprising the amino acid sequence of SEQ ID NO: 88 and a light chain variable region comprising the amino acid sequence of SEQ ID NO: 89.

An antibody or a fragment thereof comprising a heavy chain variable region comprising the amino acid sequence of SEQ ID NO: 90 and a light chain variable region comprising the amino acid sequence of SEQ ID NO: 91.

An antibody or a fragment thereof comprising a heavy chain variable region comprising the amino acid sequence of SEQ ID NO: 92 and a light chain variable region comprising the amino acid sequence of SEQ ID NO: 93.

An antibody or a fragment thereof comprising a heavy chain variable region comprising the amino acid sequence of SEQ ID NO: 94 and a light chain variable region comprising the amino acid sequence of SEQ ID NO: 95.

An antibody or a fragment thereof comprising a heavy chain variable region comprising the amino acid sequence of SEQ ID NO: 96 and a light chain variable region comprising the amino acid sequence of SEQ ID NO: 97.

An antibody or a fragment thereof comprising a heavy chain variable region comprising the amino acid sequence of SEQ ID NO: 98 and a light chain variable region comprising the amino acid sequence of SEQ ID NO: 99.

An antibody or a fragment thereof comprising a heavy chain variable region comprising the amino acid sequence of SEQ ID NO: 100 and a light chain variable region comprising the amino acid sequence of SEQ ID NO: 101.

An antibody or a fragment thereof comprising a heavy chain variable region comprising the amino acid sequence of SEQ ID NO: 102 and a light chain variable region comprising the amino acid sequence of SEQ ID NO: 103.

An antibody or a fragment thereof comprising a heavy chain variable region comprising the amino acid sequence of SEQ ID NO: 104 and a light chain variable region comprising the amino acid sequence of SEQ ID NO: 105.

An antibody or a fragment thereof comprising a heavy chain variable region comprising the amino acid sequence of SEQ ID NO: 106 and a light chain variable region comprising the amino acid sequence of SEQ ID NO: 107.

An antibody or a fragment thereof comprising a heavy chain variable region comprising the amino acid sequence of SEQ ID NO: 108 and a light chain variable region comprising the amino acid sequence of SEQ ID NO: 109.

An antibody or a fragment thereof comprising a heavy chain variable region comprising the amino acid sequence of SEQ ID NO: 110 and a light chain variable region comprising the amino acid sequence of SEQ ID NO: 111.

An antibody or a fragment thereof comprising a heavy chain variable region comprising the amino acid sequence of SEQ ID NO: 112 and a light chain variable region comprising the amino acid sequence of SEQ ID NO: 113.

An antibody or a fragment thereof comprising a heavy chain variable region comprising the amino acid sequence of SEQ ID NO: 114 and a light chain variable region comprising the amino acid sequence of SEQ ID NO: 115.

An antibody or a fragment thereof comprising a heavy chain variable region comprising the amino acid sequence of SEQ ID NO: 116 and a light chain variable region comprising the amino acid sequence of SEQ ID NO: 117.

An antibody or a fragment thereof comprising a heavy chain variable region comprising the amino acid sequence of SEQ ID NO: 118 and a light chain variable region comprising the amino acid sequence of SEQ ID NO: 119.

An antibody or a fragment thereof comprising a heavy chain variable region comprising the amino acid sequence of SEQ ID NO: 120 and a light chain variable region comprising the amino acid sequence of SEQ ID NO: 121.

An antibody or a fragment thereof comprising a heavy chain variable region comprising the amino acid sequence of SEQ ID NO: 122 and a light chain variable region comprising the amino acid sequence of SEQ ID NO: 123.

An antibody or a fragment thereof comprising a heavy chain variable region comprising the amino acid sequence of SEQ ID NO: 124 and a light chain variable region comprising the amino acid sequence of SEQ ID NO: 125.

An antibody or a fragment thereof comprising a heavy chain variable region comprising the amino acid sequence of SEQ ID NO: 126 and a light chain variable region comprising the amino acid sequence of SEQ ID NO: 127.

An antibody or a fragment thereof comprising a heavy chain variable region comprising the amino acid sequence of SEQ ID NO: 128 and a light chain variable region comprising the amino acid sequence of SEQ ID NO: 129.

An antibody or a fragment thereof comprising a heavy chain variable region comprising the amino acid sequence of SEQ ID NO: 130 and a light chain variable region comprising the amino acid sequence of SEQ ID NO: 131.

An antibody or a fragment thereof comprising a heavy chain variable region comprising the amino acid sequence of SEQ ID NO: 132 and a light chain variable region comprising the amino acid sequence of SEQ ID NO: 133.

An antibody or a fragment thereof comprising a heavy chain variable region comprising the amino acid sequence of SEQ ID NO: 300 and a light chain variable region comprising the amino acid sequence of SEQ ID NO: 304.

In Examples mentioned later, conjugates with a regulator of microtubule were prepared using the polyclonal antibody or the monoclonal antibody against the full-length CAPRIN-1 protein or a polypeptide of a partial region thereof expressed on the cell membrane surface of cancer cells, and verified to have a strong antitumor effect.

### <Benzodiazepine>

Some benzodiazepines are known to exert antitumor effects by recognizing a particular DNA sequence to bind to the DNA minor groove and blocking DNA synthesis to inhibit the cell growth. In the present invention, benzodiazepine having such antitumor activity is preferably used.

Specific examples of benzodiazepine having antitumor activity include pyrrolobenzodiazepine (PBD), indolynobenzodiazepine (IGN), pyridinobenzodiazepine (PDD), and isoquinolidinobenzodiazepine (IQB).

PBD has an aromatic ring A (A ring), an aromatic ring B (B ring), and a pyrrolo-ring C (C ring), and the position of N10-N11 is either imine (N=C), carbinolamine (NH-CH(OH)), or carbinolamine methyl ether (NH-CH(OMe)) in the B ring. Also, PBD can be in the form of a C2-unsaturated PBD dimer, a C2-aryl-substituted PBD dimer, or polypyrrole-PBD. PBD is capable of recognizing and binding to a particular sequence of DNA. A preferable sequence is 5'PuGPu3'.

Specific examples of PBD include Anthramycin, which is an antitumor antibiotics, and analogs thereof. Examples of Anthramycin analogs include Abbeymycin, Chicamycin, Mazethramycin, Neothramycin A, Meothramycin B, Porothramycin, Prothracarcin, Sibanomicin (DC-102), Sibiromycin, Tomamycin, Didehydroanhydroanthramycin, Spadicomycin, and DC-81.

In addition, specific examples of PBD include the compounds described in "Mantaj, J. et al., 2016, Angew. Chem. Int. Ed., 55,2-29; D. Antonow and D. Thurston, Chem. Rev., 2011," "Leanna et al., J. Med. Chem., 2020, 63, 9603-9622," "Jhon et al., Expert Opinion on Biological Therapy, Vol. 21, 2021, 931-943," and "Julia et al., Angew Chem. Int. Ed. Engl., Jan 9, 2017; 56 (2): 462-488."

Specific examples of PBD-linker conjugated compounds include compounds described in WO 1993/018045, WO 2000/046228, WO 2002/083682, WO 2005/040170, WO 2005/110423, WO 2005/085251, WO 2006/135687, WO 2010/043880, WO 2013/055990, WO 2013/055993, WO 2014/057072, WO 2014/159981, WO 2014/057120, WO 2015/052532, WO 2015/181559, WO 2017/020972, WO 2017/059289, WO 2017/137555, WO 2017/137556, WO 2017/186894, WO 2018/031662, WO 2018/069490, WO 2018/091646, WO 2018/146188, WO 2018/146189, WO 2018/18234, WO 2018/192944, WO 2019/034764, WO 2019/065964, WO 2019/096788, WO 2019/104289, WO 2019/126691, WO 2019/224340, WO 2020/006722, WO 2020/079229, WO 2020/079239, WO 2020/100954, WO 2020/141923, WO 2020/152462, WO 2020/1964474, WO 2020/196712, WO 2021/137646, WO 2022/063853, WO 2016/083468, WO 2014/057073, WO 2014/057113, WO 2014/057114, WO 2014/057115, WO 2014/057117, WO 2014/057118, WO 2014/057119, WO 2014/057120, WO 2014/057122, U.S. Patent No. 4316900, WO 2020/245283, WO 2019/197602, WO 2004/043963, WO 2005/085260, WO 2006/111759, WO 2010/010347, WO 2010/043877, WO 2011/128650, WO 2011/130598, WO 2011/130613, WO 2011/130616, WO 2013/041606, WO 2013/053871, WO 2013/053873, WO 2013/055987, WO 2013/053872, WO 2013/164593, WO 2013/164592, WO 2014/096368, WO 2014/140862, WO 2014/140174, WO 2015/052322, WO 2015/052532, WO 2015/052533, WO 2015/052534, WO 2015/052535, WO 2016/038383, WO 2016/037644, WO 2016/044560, WO 2017/137553, WO 2017/129652, WO 2017/223275, WO 2018/146199, and WO 2018/182341.

Examples of IGN and IGN-linker conjugated compounds include compounds described in WO 2017/015495, WO 2017/015496, WO 2012/128868, WO 2012/112687, WO 2012/112708, WO 2016/036801, WO 2017/015502, WO 2012/112708, WO 2018/140435, WO 2018/195245, WO 2018/098258, WO 2010/091150, WO 2016/036804, WO 2019/133652, WO 2020/102051, WO 2020/102053, and WO 2020/205564.

Examples of PDD and PDD-linker conjugated compounds include compounds described in WO 2012/152915, WO 2015/166289, WO 2017/032983, WO 2016/198869, WO 2017/194960, WO 2019/043417, WO 2020/049286, WO 2020/157491, and WO 2022/023735.

Examples of IQB and IQB-linker conjugated compounds include compounds described in WO 2016/149546, WO 2018/071455, WO 2018/053552, WO 2017/011803, WO 2017/091615, U.S. Patent No. 9974864, U.S. Patent No. 9504694, and U.S. Patent No. 10350218.

Benzodiazepine used in the present invention may be a monomer or a dimer. In the case of a dimer, benzodiazepine may be a homodimer or a heterodimer with another antitumor compound. The dimer molecules may bind to each other through carbon at position 8, 7, or 2 of benzodiazepine.

Benzodiazepine that is preferably used in the present invention is DSB-120, SJG-136 (SG2000), DC-81, DSB-120, SJG-136, SG2057, SG2202, SG2285, SGD-1882, SGD-1910, SG3199, SG3249, SG2219, IMGN779, IMGN632, (S)-N-(4-aminophenyl)-4-(4-(4-(4-((2-methoxy-12-oxo-6a,7,8,9,10,12-hexahydrobenzo[e]pyrido[1,2-a][1,4]diazepin-3-yl)oxy)butanamide)-1-methyl-1H-pyrrole-2-carboxamide)phenyl)-1-methyl-1H-pyrrole-2-carboxamide, D211, D221, D231, GWL-78, KMR-28-39, or a derivative of any thereof.

The derivative is preferably a derivative of the compound comprising a linker bound thereto. Specific examples include Tesirine (SG3249 derivative), Talirine (SGD-1910 derivative), SG3364, SG3227, SG3140 (MC-Phe-Lys-PAB-SG2057), SG3170, SG3203 (MC-Phe-Lys-PAB-SG2057), SG3231, SG3400, SG3376, DGN642, DGN549, FGX5-67, FGX-2-62, and FGX11-38.

### <Conjugate of anti-CAPRIN-1 antibody and benzodiazepine>

In the present invention, the mode of binding between the anti-CAPRIN-1 antibody and benzodiazepine in the conjugate of the anti-CAPRIN-1 antibody and benzodiazepine is not particularly limited as long as it allows antitumor activity against a cancer to be maintained. The mode of binding preferably has a linker structure formed between the anti-CAPRIN-1 antibody and benzodiazepine.

In this context, the linker means a compound capable of linking the anti-CAPRIN-1 antibody to benzodiazepine. Any of various linkers known in the art may be used, or an appropriate chemical modification to the structure of the activator may be used for directly binding the antibody to the activator.

The details of the type of the linker and the binding method can be basically in accordance with a method known in the art (see, for example, Greg T. Hermanson Bioconjugate Techniques, Third Edition, WO 2004/010957, and WO 2014/012479).

In an embodiment of the present invention, examples of reactive groups attached to the anti-CAPRIN-1 antibody, benzodiazepine, and the linker include the following.

Examples of the reactive group attached to the amino acid sequence of the antibody or a glycoprotein modifying an amino acid include primary amine (ε-amino group), carboxyl, thiol (sulfhydryl), carbonyl (ketone or aldehyde), and hydroxyl unless a special chemical modification is made. The primary amine exists at the N-terminus of a polypeptide or the side chain of a lysine residue and is positively charged under physiological conditions. The primary amine usually exists outside of the protein and can therefore be used in binding without denaturing the structure of the protein. The carboxyl exists at the C-terminus of a polypeptide or the side chain of aspartic acid or glutamic acid. The sulfhydryl exists at the side chain of cysteine and forms a disulfide bond that maintains the higher-order structure of the protein. The ketone or aldehyde group is generated in a glycoprotein by the oxidation of glycosyl with sodium metaperiodate.

The conjugate of the present invention is prepared by binding benzodiazepine to a linker bound to the reactive group of the antibody, binding benzodiazepine to the linker bound to benzodiazepine, or directly binding benzodiazepine to the antibody.

Specific examples of the reactive groups attached to PBD and the linker include the following.

Reactive groups capable of reacting with the amine: N-hydroxysuccinimide (NHS) ester, imide ester, pentafluorophenyl ester, hydroxymethyl phosphine, isothiocyanate, isocyanate, acyl azide, N-hydroxyl ester, sulfonyl chloride, aldehyde, glyoxal, epoxide, oxirane, carbonate, aryl, carbodiimide, and carboxylic anhydride.

Reactive groups capable of reacting with the carboxyl and the amine: carbodiimide, diazoalkane, diazoacetyl compounds, and carbonyldiimidazole.

Reactive groups capable of reacting with the thiol: maleimide, haloacetamide, pyridyl disulfide, thiosulfone, vinyl sulfone, haloacetyl, aziridine, acryloyl, and aryl.

Reactive groups capable of reacting with the aldehyde: hydrazide and alkoxyamine. Reactive groups capable of reacting with the hydroxyl: epoxy, oxirane, carbonyldiimidazole, N,N'-disuccinimidyl carbonate, N-hydroxysuccinimidyl chloroformate, and isocyanate.

Reactive group capable of reacting with the hydroxyl: isocyanate.

Photoreactive reactive groups: diaziridine, aryl azide, aryl, benzophenol, and diazo compounds.

Specific examples of linkers having the reactive groups include the following.

Linkers having the same reactive group ends: linkers having N-hydroxysuccinimide ester as a reactive group (e.g., Disuccinimidyl Glutarate (DSG), Disuccinimidyl Suberate (DSS), Bis(sulfosuccinimidyl)Suberate (BS3), Tris-(Succinimidyl)Aminotriacetate (TSAT), PEGylated Bis(Ssulfosuccinimidyl)Suberate (BS(PEG)5, BS(PEG)9), Dithiobis (Succinimidyl Propionate) (DSP), 3,3'-dithiobis(sulfosuccinimidyl propionate) (DTSSP), ethylene glycol bis(succinimidyl succinate) (EGS), Sulfo-ethylene glycol bis(succinimidyl succinate) (Sulfo-EGS), Dimethyl adipimidate·2HCl (DMA), Dimethyl pimelimidate·2HCl (DMP), Dimethyl suberimidate·2HCl (DMS), Dimethyl 3,3'-dithiobispropionimidate·2HCl (DTBP), 1,5-difluoro-2,4-dinitrobenzene (DFDNB), Disuccinimidyl tartrate (DST), Bis[2-(Succinimidooxycarbonyloxy)ethyl]Sulfone (BSOCOES), and a linker having maleimide as a reactive group (e.g., Bismaleimidoethane (BMOE), 1,4-bismaleimidobutane (BMB), Bismaleimidohexane (BMH), Tris(2-maleimidothyl)amine (TMEA), 1,8-bismaleimido-(PEG)2 (BM(PEG)2), 1,8-bismaleimido-(PEG)3 (BM(PEG)3), and Dithiobismaleimidoethane (DTME)).

Linkers having different reactive group ends: linkers having NHS ester and maleimide as reactive groups (e.g., AMAS, BMPS, GMBS, Sulfo-MBS, MBS, Sulfo-MBS, SMCC, Sulfo-SMCC, EMCS, Sulfo-EMCS, SMPB, Sulfo-SMPB, SMPH, LC-SMCC, Sulfo-KMUS, SM(PEG)2, SM(PEG)4, SM(PEG)6, SM(PEG)8, SM(PEG)12, and SM(PEG)24); linkers having NHS ester and pyridyldithiol as reactive groups (e.g., SPDP, LC-SPDP, Sulfo-LC-SPDP, SMPT, (PEG)4-SPDP, and (PEG)12-SPDP); linkers having NHS ester and haloacetyl as reactive groups (e.g., SIA, SBAP, SIAB, and Sulfo-SIAB); linkers having NHS ester and aryl azide as reactive groups (e.g., ANB-NOS, Sulfo-SANPAH, and ATFB); linkers having NHS ester and diaziridine as reactive groups (e.g., SDA, Sulfo-SDA, LC-SDA, SDAD, and Sulfo-SDAD); linkers having carbodiimide as reactive groups (e.g., DCC, EDC, EDAC, NHS, and Sulfo-NHS); linkers having maleimide and hydrazide as reactive groups (e.g., BMPH, EMCH, MPBH, and KMUH); linkers having pyridyldithiol and hydrazide as a reactive group (e.g., PDPH); linkers having isocyanate and maleimide as reactive groups (e.g., PMPI); and linkers having NHS ester and psoralen as reactive groups (e.g., SPB).

Other linkers: linkers containing polypeptide, for example, Fmoc-Ala-Ala-Asn-PAB, Fmoc-Ala-Ala-Asn(Trt)-PAB, Fmoc-PEG3-Ala-Ala-Asn(Trt)-PAB, Fmoc-PEG4-Ala-Ala-Asn(Trt)-PAB, Fmoc-Ala-Ala-Asn-PAB-PNP, Fmoc-Ala-Ala-Asn(Trt)-PAB-PNP, Fmoc-PEG3-Ala-Ala-Asn(Trt)-PAB-PNP, Azide-PEG4-Ala-Ala-Asn(Trt)-PAB-PNP, Mal-PEG4-Ala-Ala-Asn(Trt)-PAB-PNP, Fmoc-Val-Cit-PAB-OH, Val-Cit-PAB-OH, Fmoc-Val-Cit-PAB-PNP, MC-Val-Cit-PAB, and MC-Val-Cit-PAB-PNP.

Also, Bis-PEG-acid, PEG Acid (e.g., Acid-PEG-TEMPO, Amino-PEG-acid, Amino-PEG-CH₂CO₂H, Aminoxy-PEG-acid, Azido-PEG-acid, Carboxy-PEG-sulfonic acid, Fmoc-N-amido-PEG-acid, Fmoc-N-amido-PEG-CH₂CO₂H, Fmoc-aminooxy-PEG-acid, Hydroxy-PEG-acid, Hydroxy-PEG-CH₂CO₂H, m-PEG-acid, m-PEG-(CH₂)₃-acid, Methoxytrityl-N-PEG-acid, N-methyl-N-(t-Boc)-PEG-acid, Propargyl-PEG-acid, Propargyl-PEG-CH₂CO₂H, Propargyl-PEG-(CH₂)₃-acid, t-Boc-N-amido-PEG-acid, t-Boc-N-amido-PEG-CH₂CO₂H, t-Boc-Aminooxy-PEG-acid, Acid-PEG-PFP ester, Miscellaneous PEG acid), PEG PFP ester (e.g., Acid-PEG-PFP ester, Bis-PEG-PFP ester), Bis-PEG-NHS, PEG Aldehyde (e.g., m-PEG-aldehyde, m-PEG-benzaldehyde, Ald-PEG-acid, Ald-PEG-amine, Ald-PEG-azide, Ald-PEG-NH-Boc, Ald-PEG-NHS ester, Ald-PEG-TFP ester, Ald-PEG-t-butyl ester), PEG Tosylate (e.g., Azido-PEG-Tos, Hydroxy-PEG-Tos, m-PEG-Tos, t-Boc-Aminooxy-PEG-Tos, Trifluoroethyl-PEG-Tos, Tos-PEG-acid, Tos-PEG-CH₂CO₂H, Tos-PEG-alkyne, Tos-PEG-t-butyl ester, Tos-PEG-CH₂CO₂tBu, Tos-PEG-Tos, S-acetyl-PEG6-Tos, N-Tos-N-(t-butoxycarbonyl)-aminooxy-PEG4-Tos, Ms-PEG-Ms, Ms-PEG-t-butyl ester, PEG-Ms, Propargyl-PEG-Ms), Boc-PEG (e.g., Amino-PEG-t-Boc-Hydrazide, Azido-PEG-t-Boc-Hydrazide, Boc-NH-PEG-NH-Boc, Bromoacetamido-PEG-Boc-amine, m-PEG-ONHBoc, Mal-Alkyl-t-Boc-amine, N-Boc-PEG-alcohol, N-Boc-PEG-bromide, N-methyl-N-(t-Boc)-PEG-acid, t-Boc-N-amido-PEG-acid, t-Boc-N-amido-PEG-CH₂CO₂H, t-Boc-N-Amido-PEG-amine, t-Boc-N-amido-PEG-azide, t-Boc-N-amido-PEG-NHS ester, t-Boc-N-amido-PEG-sulfonic acid), PEG NHS ester (e.g., Acid-PEG-NHS ester, Azido-PEG-NHS ester, Bis-PEG-NHS, Fmoc-PEG-NHS ester, m-PEG-NHS ester, m-PEG-NHS Carbonate, Mal-PEG-NHS ester, Propargyl-PEG-NHS ester, t-Boc-N-amido-PEG-NHS ester, t-Butoxycarbonyl-PEG-NHS ester), Fmoc-PEG (e.g., Fmoc-N-amido-PEG-acid, Fmoc-NH-PEG-CH₂CO₂H, Fmoc-PEG-NHS ester), Biotin PEG (e.g., Biotin PEG-acid, Biotin PEG-alcohol, Biotin PEG-alkyne, Biotin PEG-amine, Biotin PEG-azide, Biotin PEG-DBCO, Biotin PEG-hydrazide, Biotin-PEG-Mal, Biotin-PEG-NHS, Biotin-EDA-PEG-NHS, Biotin-PEG-oxyamine, Biotin-PEG-PFP, Biotin-EDA-PEG-PFP, Biotin-PEG-Tetrazine, Biotin-PEG-TFP, Azide-SS-biotin, Biotin-PEG3-SS-azide, DBCO-S-S-PEG3-Biotin, Dde Biotin-PEG4-Alkyne, Dde Biotin-PEG4-Azide, Dde Biotin-PEG4-DBCO, Diazo Biotin-PEG3-Alkyne, Diazo Biotin-PEG3-Azide, Diazo Biotin-PEG3-DBCO, Diol Biotin-PEG3-Alkyne, Diol Biotin-PEG3-Azide, PC Biotin-PEG3-Alkyne, PC-Biotin-PEG4-PEG4-Alkyne, PC-Biotin-PEG4-PEG4-Alkyne, PC Biotin-PEG3-Azide, PC-Biotin-PEG4-PEG3-Azide, PC-Biotin-PEG4-NHS carbonate, PC DBCO-PEG3-Biotin, WSPC Biotin-PEG3-DBCO, Fmoc-Lys (biotin-PEG)-OH, Fmoc-N-amido-(PEG-biotin)-acid, TAMRA-Azide-PEG-Biotin), PEG Phosphonate, Aminooxy PEG (e.g., Aminooxy-PEG-acid, Aminooxy-PEG-alcohol, Aminooxy-PEG-azide, Aminooxy-PEG-bromide, Aminooxy-PEG-methane, Aminooxy-PEG-Propargyl, Aminooxy-PEG-t-butyl ester, Aminooxy-PEG-Thiol, Bis-(Aminooxy)-PEG, t-Boc-Aminooxy-PEG-acid, t-Boc-Aminooxy-PEG-alcohol, t-Boc-Aminooxy-PEG-amine, t-Boc-Aminooxy-PEG-Azide, t-Boc-Aminooxy-PEG-Bromide, t-Boc-aminooxy-PEG-Methane, t-Boc-aminooxy-PEG-Propargyl, t-Boc-aminooxy-PEG-S-Ac, t-Boc-Aminooxy-PEG-Thiol, t-Boc-Aminooxy-PEG-Tos, Fmoc-aminooxy-PEG-acid, Trifluoroethyl-PEG-Aminooxy), Alkyne PEG (e.g., endo-BCN-PEG, exo-BCN-PEG, Propargyl-PEG-acid, Propargyl-PEG-CH₂CO₂H, Propargyl-PEG-(CH₂)₃-acid, Propargyl-PEG-(CH₂)₃-methyl ester, Propargyl-PEG-Acrylate, Propargyl-PEG-alcohol, Propargyl-PEG-amine, Propargyl-PEG-methylamine, Aminooxy-PEG-Propargyl, Propargyl-PEG-azide, Propargyl-PEG-bromide, Propargyl-PEG-Maleimide, Propargyl-PEG-Ms, Propargyl-PEG-NHS ester, Propargyl-PEG-sulfonic acid, Propargyl-PEG-t-butyl ester, Propargyl-PEG-CH₂CO₂tBu, Propargyl-PEG-thiol, Propargyl-PEG-5-nitrophenyl carbonate, t-Boc-aminooxy-PEG-Propargyl, Bis-Propargyl-PEG, m-PEG-Propargyl), Azido PEG (e.g., Azido-PEG-acid, Azido-PEG-CH₂CO₂H, Azido-PEG-(CH₂)₃-methyl ester, Azido-PEG-Acrylate, Azido-PEG-alcohol, Azido-PEG-(CH₂)₃OH, Azido-PEG-amine, Azido-PEG-azide, Azido-PEG-Maleimide, Azido-PEG-methylamine, Azido-PEG-methyl ester, Azido-PEG-NHS ester, Azido-PEG-CH₂CO₂-NHS, Azido-PEG-oxazolidin-2-one, Azido-PEG-PFP ester, Azido-PEG-phosphonic acid, Azido-PEG-phosphonic acid ethyl ester, Azido-PEG-sulfonic acid, Azido-PEG-t-Boc-Hydrazide, Azido-PEG-t-butyl ester, Azido-PEG-CH₂CO₂-t-butyl ester, Azido-PEG-TFP ester, Azido-PEG-Tos, Aminooxy-PEG-azide, Bromo-PEG-azide, Bromoacetamido-PEG-azide, Carboxyrhodamine 110-PEG-Azide, Isothiocyanato-PEG-Azide, Isothiocyanato-PEG-Azide, m-PEG-azide, Propargyl-PEG-azide, TAMRA-PEG-Azide, t-Boc-N-Amido-PEG-Azide, t-Boc-Aminooxy-PEG-Azide, Thiol-PEG-Azide, Trifluoroethyl-PEG-Azide, Azido-PEG-amino acid, Azido-PEG4-4-nitrophenyl carbonate, S-Acetyl-PEG3-Azido, Azide, Trityl-PEG10-Azide), Alkyne PEG, DBCO-PEG, BCN-PEG, Propargyl-PEG, Bis-PEG-acid, Bis-PEG-NHS, Bis-PEG-PFP, Bis-Propargyl-PEG, Amine-PEG-Amine, Azido-PEG-azide, Bromo-PEG, or Mal PEG may be used.

According to another embodiment, a conjugate that is expected to improve the kinetics *in vivo* can be obtained with the use of the polyethylene glycol (PEG) described in WO 2015/057699 or WO 2017/165851.

The linker between the anti-CAPRIN-1 antibody and benzodiazepine may be composed of a single linker or composed of a plurality of linkers.

A method for preparing the conjugate of the anti-CAPRIN-1 antibody and benzodiazepine includes a method which involves binding benzodiazepine using a ε-amino group at the lysine side chain of the antibody and a method which involves binding benzodiazepine using thiol formed by the reduction treatment of a cysteine residue constituting the disulfide bond of the antibody.

In the case of using a ε-amino group at the lysine residue of the antibody, for example, a method is used which involves reacting active ester (e.g., N-hydroxysuccinimide ester) therewith to form an amide bond. In this case, since the antibody contains many lysine residues, the binding reaction proceeds nonspecifically.

In the case of using thiol constituting a disulfide bond present at the cysteine side chain of the antibody, a method is used which involves forming thiol from the disulfide bond on the antibody using a reducing agent such as mercaptoethanol, and reacting the thiol with maleimide or α-haloamide. For example, a method using sulfone phenyloxadiazole, a 4-cyanoethynyloxy derivative, or the like is used for stabilizing a thiol-mediated bond. These bonds are stable for a longer time than the bond based on the conjugate addition reaction of cysteine with maleimide. Also, a linker having an amino group near an imide group can be used because an imide ring with a thiol group added to maleimide is opened by hydrolysis so that stability is improved owing to the resulting amide bond. In the antibody, the thiols of cysteines form a disulfide bond. Thus, an alternative method involves binding benzodiazepine, etc. to a site between two thiols via the thiols. As an example, a cross-linked bond may be formed using a linker having two disulfide bond sites that can be formed from an amide group having two sulfones at the β position, or dibromomaleimide.

The conjugate of the present invention can be obtained by a method using, for example, the THIOMAB^{™} technique, which is a method capable of introducing a determined number of thiol groups at a particular site of an antibody (see Nature Biotechnology 26, 925-932 (2008)), a method involving the use of the ThioBridge^{™} technique (see Methods Mol. Biol. 2078: 113-129 (2020)), or the RESPECT^{™} technique (see MAbs.9 (6): 907-915 (2017)).

The conjugate of the present invention can be formed, for example, by reducing the antibody using a reducing agent dithiothreitol (DTT) in a phosphate buffer to obtain an antibody having a reactive thiol group, which is then conjugated with benzodiazepine. The conjugate can be obtained by adding a thiol group to primary amine at the lysine residue of the antibody by the introduction of a Traut's reagent (2-iminothiolane or N-succinimidyl S-acetylthioacetate (SATA)), instead of the method using a reducing agent.

The amount of the thiol added to the antibody can be determined, for example, by mixing a sample solution containing 5,5'-dithiobis(2-nitrobenzoic acid) (DTNB) and an SH group with a phosphate buffer solution (pH 8.0) and distilled water, adding a solution of DTNB dissolved in a phosphate buffer, a Good's buffer, or a Tris buffer to the mixture, and incubating the resulting mixture for a given time, followed by the measurement of absorbance at 412 nm (see G.L. Ellman, Arch. Biochem. Biophys., 82, 70 (1959)).

The thiol group added by the cleavage of the disulfide bond of the antibody through reduction treatment is preferably treated (capped) in order to prevent the formation of a disulfide bond again. The capping can employ, for example, N-ethylmaleimide (NEM) or 2-iodoacetamide (IAA).

The conjugate can be formed by binding benzodiazepine to the antibody using the thiol group added to the antibody in accordance with a method known in the art. Specifically, the binding can be carried out using, for example, a linker reagent having a maleimide group or a bromoacetamide group as a linker reagent specifically binding to the thiol group of the reduced antibody. For example, N-succinimidyl-4-(N-maleimidomethyl)-cyclohexane-1-carboxylate (SMCC) is used as the linker having a maleimide group. In this case, the N-succinimide group of SMCC can form an amide bond with an amino group present in benzodiazepine to obtain the conjugate.

In another embodiment, an amide bond is first formed at an amino group present in the activator using SMCC. Then, the maleimide group of the SMCC bound with benzodiazepine can be reacted with the thiol group added to the antibody to obtain the conjugate.

In an alternative embodiment, the conjugate of the antibody and benzodiazepine may be formed using two linkers. For example, a primary amino group present at the lysine residue of the antibody is bound to the N-succinimide group of SATA (N-succinimidyl-S-acetylthioacetate) via an amide bond to add a thiol group to the antibody. SMCC is reacted with benzodiazepine containing an amino group or with benzodiazepine having an amino group added thereto according to an ordinary method to form an amide bond with the N-succinimide group of the SMCC. The maleimide group of the SMCC bound with benzodiazepine can be reacted with the thiol group of the SATA bound with the antibody to obtain the conjugate.

In a further alternative embodiment, examples of the preparation of the conjugate of the antibody and benzodiazepine include a method using maleimidocaproyl-valine-citrulline-p-aminobenzyloxycarbonyl (MC-Val-Cit-PAB) as a linker. MC-val-Cit-PAB (mc-vc-PAB) is a linker cleavable by intracellular protease (e.g., cathepsin B). A thiol group is added to the antibody dissolved in a phosphate buffer using DTT or the like. Meanwhile, benzodiazepine having an amino group is reacted with the benzyloxycarbonyl (PAB) in mc-Val-Cit-PAB to prepare benzodiazepine bound with mc-val-Cit-PAB, which can then be reacted with the thiol-added antibody mentioned above to obtain the conjugate.

In a further alternative embodiment, SATA is bound to a primary amino group at the lysine residue of the antibody to add a thiol group thereto. Meanwhile, succinimidyl 3-(2-pyridyldithio)propionate (SPDP) is reacted with benzodiazepine having an amino group to form an amide bond with the N-succinimide group of the SPDP.

A linker used in the present invention is cleavable in cells, and a substance having antitumor activity, which comprises benzodiazepine or benzodiazepine and a part of the linker, is liberated in cells. For example, a linker is cleaved by intracellular peptidase or protease. Preferably, a linker is cleaved by lysosome, endosome protease, cathepsin B, cathepsin D, or plasmin. An example is a linker comprising a polypeptide cleavable by cathepsin B (Val-Cit, Phe-Leu or Gly-Phe-Leu-Gly). More particularly, the linker described in U.S. Patent 6,214,345 can be used.

In a further alternative embodiment, for example, the linker comprising glucuronic acid (preferably β-D-glucuronide) described in WO 2007/0711968 can be used as a means for improving stability, solubility, and metabolism of the conjugate of the present invention in blood and binding affinity of benzodiazepine to the anti-CAPRIN-1 antibody. Alternatively, the means described in WO 2013/173337, WO 2015/095755, WO 2015/123679, or WO 2018/031690 can also be used.

In a further alternative embodiment, it is possible to bind benzodiazepine in a site-directed manner to the anti-CAPRIN-1 antibody using the means described in, for example, WO 2006/65533 or WO 2018/160683.

In a further alternative embodiment, it is possible to obtain a conjugate of the anti-CAPRIN-1 antibody and two or more drugs including benzodiazepine in accordance with the method described in WO 2018/112253.

In order to obtain a composition comprising the conjugate of the anti-CAPRIN-1 antibody and benzodiazepine of the present invention, for example, a peak fraction of a higher molecular weight as compared with the antibody before the binding of the linker can be separated by the application of gel filtration chromatography or the like. In order to detect the mass of the conjugate while maintaining the intact bivalent antibody, for example, the method described in WO 2013/049410 can be employed.

The number of molecules of the bound benzodiazepine per antibody molecule in the conjugate of the anti-CAPRIN-1 antibody and benzodiazepine of the present invention can be quantified in accordance with methods known in the art, such as mass spectrometry, ELISA, electrophoresis, or chromatography such as HPLC.

### <Antitumor effect of conjugate>

The conjugate of the anti-CAPRIN-1 antibody and benzodiazepine of the present invention has antitumor activity *in vitro* or *in vivo.* The term "antitumor activity" refers to reduction, elimination, growth inhibition, apoptosis, necrosis, or killing of target cancer cells. Accordingly, the antitumor effects of the conjugate of the present invention can be determined by examining the antitumor activity against a cancer.

The antitumor effects *in vivo* can be evaluated by: administering the conjugate to an organism having a cancer; and examining the size of the tumor over time via measuring the size of the tumor after the administration. The antitumor effect of the present invention can also be evaluated by examining a survival rate. Alternatively, the antitumor effect of the present invention may be evaluated by examining the ability to produce a cytokine or a chemokine. The antitumor effect of the conjugate of the present invention can be further determined by examining the prevention of a cancer, the prevention of metastasis, or the prevention of recurrence.

The conjugate of the present invention is expected to have a stronger antitumor effect, the higher binding affinity for the CAPRIN-1 protein on cancer cell surface the conjugate has. Its association constant (affinity constant) Ka (kon/koff) is preferably at least 10⁷ M⁻¹, at least 10⁸ M⁻¹, at least 5 x 10⁸ M⁻¹, at least 10⁹ M⁻¹, at least 5 x 10⁹ M⁻¹, at least 10¹⁰ M⁻¹, at least 5 x 10¹⁰ M⁻¹, at least 10¹¹ M⁻¹, at least 5 x 10¹¹ M⁻¹, at least 10¹² M⁻¹, or at least 10¹³ M⁻¹.

The ability of the conjugate of the present invention to bind to CAPRIN-1 can be identified through the use of binding assay using, for example, surface plasmon resonance (SPR), ELISA, Western blot, immunofluorescence, or flow cytometry.

The conjugate of the present invention enhances the antitumor effect as compared with the anti-CAPRIN-1 antibody alone, as mentioned above. The rate of the enhancement is preferably 30% or more, more preferably 40% or more, further preferably 50% or more, still further preferably 55% or more, even further preferably 60% or more, furthermore preferably 65% or more, and most preferably 70% or more. The rate of enhancement in antitumor effect by the conjugate of the present invention with respect to the anti-CAPRIN-1 antibody alone can be calculated by administering their respective effective amounts to cancer-bearing mice under the same conditions, and comparing tumor volumes 10 days or later after the start of the administration.

### <Pharmaceutical composition and method for treating and/or preventing cancer>

The target of the pharmaceutical composition for the treatment and/or prevention of a cancer of the present invention is not particularly limited as long as the target is cancer (cells) expressing the CAPRIN-1 protein.

The terms "tumor" and "cancer" used in the present specification mean malignant neoplasm and are used interchangeably with each other.

The cancer targeted in the present invention may be any cancer expressing the CAPRIN-1 protein on the cell membrane surface. The cancer is preferably breast cancer, kidney cancer, pancreatic cancer, colorectal cancer, lung cancer, brain tumor, stomach cancer, uterine cancer, ovary cancer, prostate cancer, bladder cancer, esophagus cancer, leukemia, lymphoma, liver cancer, gallbladder cancer, sarcoma, mastocytoma, melanoma, adrenal cortex cancer, Ewing's tumor, Hodgkin's lymphoma, mesothelioma, multiple myeloma, testicle cancer, thyroid cancer, head and neck cancer, or urothelial carcinoma as mentioned above.

More specifically, examples of these cancers include, but are not limited to, breast adenocarcinoma, complex-type breast adenocarcinoma, malignant mixed tumor of the mammary gland, intraductal papillary adenocarcinoma, recurrent metastatic breast cancer, lung adenocarcinoma, non-small cell lung cancer (NSCLC), squamous non-small cell lung cancer, squamous cell cancer, small-cell cancer, large-cell cancer, glioma which is tumor of neuroepithelial tissue, glioblastoma, neuroblastoma, ependymoma, neuronal tumor, embryonal neuroectodermal tumor, neurilemmoma, neurofibroma, meningioma, chronic lymphocytic leukemia, lymphoma, gastrointestinal lymphoma, alimentary lymphoma, small to medium cell-type lymphoma, cecal cancer, ascending colon cancer, descending colon cancer, transverse colon cancer, sigmoid colon cancer, rectal cancer, epithelial ovarian cancer, germ cell tumor, stromal cell tumor, pancreatic ductal carcinoma, invasive pancreatic ductal carcinoma, pancreatic adenocarcinoma, metastatic adenocarcinoma, acinar cell carcinoma, adenosquamous carcinoma, giant cell tumor, intraductal papillary-mucinous neoplasm, mucinous cystic neoplasm, pancreatoblastoma, islet-cell adenoma, Frants tumor, serous cystadenocarcinoma, solid-pseudopapillary tumor, gastrinoma, glucagonoma, insulinoma, multiple endocrine neoplasia type-1 (Wermer's syndrome), nonfunctional islet cell tumor, somatostatinoma, VIPoma, uterine cervix cancer, uterine body cancer, fibrosarcoma, sarcoma of bones or joints, Ewing's sarcoma, Wilms tumor, hepatoblastoma, soft tissue sarcoma, acute leukemia, chronic leukemia, spinal cord tumor, malignant soft tissue tumor, teratoma group tumor, and head and neck cancer including hypopharynx cancer, oropharynx cancer, tongue cancer, epipharynx cancer, oral cancer, lip cancer, sinus cancer, throat cancer, and recurrent brain tumor.

The subjects (patients) to be targeted are preferably mammals, for example, mammals including primates, pet animals, livestock, and sport animals and are particularly preferably humans, dogs, and cats.

In the case of using the conjugate used in the present invention in a pharmaceutical composition, the pharmaceutical composition can be formulated by a method known to those skilled in the art. For example, the pharmaceutical composition can be used in the form of a parenteral injection of an aseptic solution or suspension with water or any other pharmaceutically acceptable liquid. For example, the conjugate may be formulated in a unit dosage form required for generally accepted pharmaceutical practice, by mixing with pharmacologically acceptable carriers or media, specifically, sterilized water, physiological saline, a plant oil, an emulsifier, a suspending agent, a surfactant, a stabilizer, a fragrance, an excipient, a binder, etc in appropriate combination. The effective amount of the active ingredient in such a preparation is determined so that an appropriate dose within the prescribed range can be achieved.

In the case of using the conjugate of the present invention in a pharmaceutical composition, the pharmaceutical composition can be formulated to comprise an arbitrary salt, a surfactant, a buffer, a sugar, and an antifreezing agent (containing some sugar) in a lyophilized state.

An aseptic composition for injection can be formulated according to conventional pharmaceutical practice using a vehicle such as injectable distilled water. Examples of aqueous solutions for injection include physiological saline, isotonic solutions containing glucose and other auxiliary agents, for example, D-sorbitol, D-mannose, D-mannitol, and sodium chloride. These solutions may be used in combination with an appropriate solubilizer, for example, an alcohol (specifically, ethanol) or a polyalcohol (e.g., propylene glycol and polyethylene glycol), or a nonionic surfactant, for example, Polysorbate 80^{™} or HCO-60. Examples of oil solutions include sesame oil and soybean oil. These solutions may be used in combination with benzyl benzoate or benzyl alcohol as a solubilizer. The solutions may be further mixed with a buffer (e.g., a phosphate buffer solution and a sodium acetate buffer solution), a soothing agent (e.g., procaine hydrochloride), a stabilizer (e.g., benzyl alcohol and phenol), and an antioxidant. The injection solutions thus prepared are usually filled into appropriate ampules. Examples of oil solutions include sesame oil and soybean oil. These solutions may be used in combination with benzyl benzoate or benzyl alcohol as a solubilizer. The solutions may be further mixed with a buffer (e.g., a phosphate buffer solution and a sodium acetate buffer solution), a soothing agent (e.g., procaine hydrochloride), a stabilizer (e.g., benzyl alcohol and phenol), and an antioxidant. The injection solutions thus prepared are usually filled into appropriate ampules.

The administration is carried out orally or parenterally, preferably parenterally. Specific examples of its dosage forms include injections, intranasal administration preparations, transpulmonary administration preparations, and percutaneous administration preparations. Examples of the injections include intravenous injection, intramuscular injection, intraperitoneal injection, subcutaneous injection, and intratumoral administration, through which the pharmaceutical composition can be administered systemically or locally.

Also, the administration method can be appropriately selected in view of the age, body weight, sex, symptoms, etc., of a patient. The dose of a pharmaceutical composition containing the antibody or a polynucleotide encoding the antibody can be selected within a range of, for example, 0.0001 to 1000 mg/kg of body weight per dose. For example, the dose can be 0.5 mg, 1 mg, 2 mg, 3 mg, 5 mg, 10 mg, 20 mg, 50 mg, 75 mg, 100 mg, 200 mg, 500 mg, or 1000 mg/kg of body weight per dose. Alternatively, the dose can be selected within a range of, for example, 0.001 to 100000 mg/body of a patient, although the dose is not necessarily limited to these numeric values.

Although the dose and the administration method vary depending on the body weight, age, sex, symptoms, etc., of a patient, those skilled in the art can appropriately select the dose and the method.

The pharmaceutical composition for the treatment and/or prevention of a cancer comprising the conjugate of the present invention as an active ingredient can be administered to a subject to treat and/or prevent the aforementioned cancer expressing CAPRIN-1 on the cell membrane surface, preferably breast cancer, kidney cancer, pancreatic cancer, colorectal cancer, lung cancer, brain tumor, stomach cancer, uterine cancer, ovary cancer, prostate cancer, bladder cancer, esophagus cancer, leukemia, lymphoma, liver cancer, gallbladder cancer, sarcoma, mastocytoma, melanoma, adrenal cortex cancer, Ewing's tumor, Hodgkin's lymphoma, mesothelioma, multiple myeloma, testicle cancer, thyroid cancer, head and neck cancer, or urothelial carcinoma.

### Examples

Hereinafter, the present invention will be specifically described with reference to Examples. However, the scope of the present invention is not intended to be limited by these specific examples.

### (Example 1) Anti-CAPRIN-1 polyclonal antibody

In order to obtain anti-CAPRIN-1 polyclonal antibodies having immunological reactivity with the CAPRIN-1 protein for use in the conjugates of the present invention, 1 mg of a human CAPRIN-1 recombinant protein of SEQ ID NO: 2 or SEQ ID NO: 4 prepared according to Example 3 of WO 2010/016526 was mixed with an equal volume of incomplete Freund's adjuvant (IFA) solution. This mixture was subcutaneously administered to each rabbit four times every 2 weeks. Then, blood was collected to obtain antiserum containing polyclonal antibodies. The obtained antiserum was purified using a protein G carrier (GE Healthcare Bio-Sciences Corp.) to obtain a polyclonal antibody against the CAPRIN-1 protein (anti-CAPRIN-1 polyclonal antibody #1). Also, the serum of a rabbit obtained without the administration of the antigen was purified using a protein G carrier in the same way as above and used as a rabbit control antibody.

The following polyclonal antibodies #2 to #6 against partial polypeptides of CAPRIN-1 were obtained in the same way as in the method for preparing the polyclonal antibody against the CAPRIN-1 protein.

Anti-CAPRIN-1 polyclonal antibody #2 against a partial CAPRIN-1 polypeptide represented by SEQ ID NO: 37 disclosed in WO 2011/096528 (SEQ ID NO: 31 of the present specification).

Anti-CAPRIN-1 polyclonal antibody #3 against a partial polypeptide represented by SEQ ID NO: 5 disclosed in WO 2013/018894 (SEQ ID NO: 32 of the present specification).

Anti-CAPRIN-1 polyclonal antibody #4 against a partial polypeptide represented by SEQ ID NO: 5 disclosed in WO 2013/125654 (SEQ ID NO: 33 of the present specification).

Anti-CAPRIN-1 polyclonal antibody #5 against a partial polypeptide represented by SEQ ID NO: 37 disclosed in WO 2011/096533 (SEQ ID NO: 34 of the present specification).

Anti-CAPRIN-1 polyclonal antibody #6 against a partial polypeptide represented by SEQ ID NO: 37 disclosed in WO 2011/096534 (SEQ ID NO: 35 of the present specification).

### (Example 2) Anti-CAPRIN-1 monoclonal antibody

The following anti-CAPRIN-1 monoclonal antibodies were used in the conjugate of the present invention.

The monoclonal antibody against CAPRIN-1 disclosed in WO 2011/096528, wherein the antibody comprises CDR1, CDR2, and CDR3 of a heavy chain variable region consisting of the amino acid sequences of SEQ ID NO: 36, SEQ ID NO: 37, and SEQ ID NO: 38, respectively, and CDR1, CDR2, and CDR3 of a light chain variable region consisting of the amino acid sequences of SEQ ID NO: 40, SEQ ID NO: 41, and SEQ ID NO: 42, respectively (e.g., an antibody comprising the amino acid sequence of a heavy chain variable region represented by SEQ ID NO: 39 comprising the CDR1, CDR2, and CDR3 of the heavy chain variable region, and the amino acid sequence of a light chain variable region represented by SEQ ID NO: 43 comprising the CDR1, CDR2, and CDR3 of the light chain variable region).

The monoclonal antibody against CAPRIN-1 disclosed in WO 2015/020212, wherein the antibody comprises CDR1, CDR2, and CDR3 of a heavy chain variable region consisting of the amino acid sequences of SEQ ID NO: 44, SEQ ID NO: 45, and SEQ ID NO: 46, respectively, and CDR1, CDR2, and CDR3 of a light chain variable region consisting of the amino acid sequences of SEQ ID NO: 48, SEQ ID NO: 49, and SEQ ID NO: 50, respectively (e.g., an antibody comprising the amino acid sequence of a heavy chain variable region represented by SEQ ID NO: 47 comprising the CDR1, CDR2, and CDR3 of the heavy chain variable region, and the amino acid sequence of a light chain variable region represented by SEQ ID NO: 51 comprising the CDR1, CDR2, and CDR3 of the light chain variable region).

The monoclonal antibody against CAPRIN-1 disclosed in WO 2011/096519, wherein the antibody comprises CDR1, CDR2, and CDR3 of a heavy chain variable region consisting of the amino acid sequences of SEQ ID NO: 52, SEQ ID NO: 53, and SEQ ID NO: 54, respectively, and CDR1, CDR2, and CDR3 of a light chain variable region consisting of the amino acid sequences of SEQ ID NO: 56, SEQ ID NO: 57, and SEQ ID NO: 58, respectively (e.g., an antibody comprising the amino acid sequence of a heavy chain variable region represented by SEQ ID NO: 55 comprising the CDR1, CDR2, and CDR3 of the heavy chain variable region, and the amino acid sequence of a light chain variable region represented by SEQ ID NO: 59 comprising the CDR1, CDR2, and CDR3 of the light chain variable region).

The monoclonal antibody against CAPRIN-1 disclosed in WO 2013/125654, wherein the antibody comprises CDR1, CDR2, and CDR3 of a heavy chain variable region consisting of the amino acid sequences of SEQ ID NO: 60, SEQ ID NO: 61, and SEQ ID NO: 62, respectively, and CDR1, CDR2, and CDR3 of a light chain variable region consisting of the amino acid sequences of SEQ ID NO: 64, SEQ ID NO: 65, and SEQ ID NO: 66, respectively (e.g., an antibody comprising the amino acid sequence of a heavy chain variable region represented by SEQ ID NO: 63 comprising the CDR1, CDR2, and CDR3 of the heavy chain variable region, and the amino acid sequence of a light chain variable region represented by SEQ ID NO: 67 comprising the CDR1, CDR2, and CDR3 of the light chain variable region).

The monoclonal antibody against CAPRIN-1 disclosed in WO 2011/096517, wherein the antibody comprises the amino acid sequence of a heavy chain variable region represented by the amino acid sequence of SEQ ID NO: 68 and the amino acid sequence of a light chain variable region represented by the amino acid sequence of SEQ ID NO: 69.

The monoclonal antibody against CAPRIN-1 disclosed in WO 2011/096528, wherein the antibody comprises the amino acid sequence of a heavy chain variable region represented by the amino acid sequence of SEQ ID NO: 70 and the amino acid sequence of a light chain variable region represented by the amino acid sequence of SEQ ID NO: 71; the antibody comprises the amino acid sequence of a heavy chain variable region represented by the amino acid sequence of SEQ ID NO: 72 and the amino acid sequence of a light chain variable region represented by the amino acid sequence of SEQ ID NO: 73; the antibody comprises the amino acid sequence of a heavy chain variable region represented by the amino acid sequence of SEQ ID NO: 74 and the amino acid sequence of a light chain variable region represented by the amino acid sequence of SEQ ID NO: 75; the antibody comprises the amino acid sequence of a heavy chain variable region represented by the amino acid sequence of SEQ ID NO: 76 and the amino acid sequence of a light chain variable region represented by the amino acid sequence of SEQ ID NO: 77; or the antibody comprises the amino acid sequence of a heavy chain variable region represented by the amino acid sequence of SEQ ID NO: 78 and the amino acid sequence of a light chain variable region represented by the amino acid sequence of SEQ ID NO: 79.

The monoclonal antibody against CAPRIN-1 disclosed in WO 2011/096533, wherein the antibody comprises the amino acid sequence of a heavy chain variable region represented by the amino acid sequence of SEQ ID NO: 80 and the amino acid sequence of a light chain variable region represented by the amino acid sequence of SEQ ID NO: 81, or the antibody comprises the amino acid sequence of a heavy chain variable region represented by the amino acid sequence of SEQ ID NO: 82 and the amino acid sequence of a light chain variable region represented by the amino acid sequence of SEQ ID NO: 83.

The monoclonal antibody against CAPRIN-1 disclosed in WO 2011/096534, wherein the antibody comprises the amino acid sequence of a heavy chain variable region represented by the amino acid sequence of SEQ ID NO: 84 and the amino acid sequence of a light chain variable region represented by the amino acid sequence of SEQ ID NO: 85, or the antibody comprises the amino acid sequence of a heavy chain variable region represented by the amino acid sequence of SEQ ID NO: 86 and the amino acid sequence of a light chain variable region represented by the amino acid sequence of SEQ ID NO: 87.

The monoclonal antibody against CAPRIN-1 disclosed in WO 2010/016526, wherein the antibody comprises the amino acid sequence of a heavy chain variable region represented by the amino acid sequence of SEQ ID NO: 88 and the amino acid sequence of a light chain variable region represented by the amino acid sequence of SEQ ID NO: 89; the antibody comprises the amino acid sequence of a heavy chain variable region represented by the amino acid sequence of SEQ ID NO: 90 and the amino acid sequence of a light chain variable region represented by the amino acid sequence of SEQ ID NO: 91; the antibody comprises the amino acid sequence of a heavy chain variable region represented by the amino acid sequence of SEQ ID NO: 92 and the amino acid sequence of a light chain variable region represented by the amino acid sequence of SEQ ID NO: 93; the antibody comprises the amino acid sequence of a heavy chain variable region represented by the amino acid sequence of SEQ ID NO: 94 and the amino acid sequence of a light chain variable region represented by the amino acid sequence of SEQ ID NO: 95; the antibody comprises the amino acid sequence of a heavy chain variable region represented by the amino acid sequence of SEQ ID NO: 96 and the amino acid sequence of a light chain variable region represented by the amino acid sequence of SEQ ID NO: 97; the antibody comprises the amino acid sequence of a heavy chain variable region represented by the amino acid sequence of SEQ ID NO: 98 and the amino acid sequence of a light chain variable region represented by the amino acid sequence of SEQ ID NO: 99; or the antibody comprises the amino acid sequence of a heavy chain variable region represented by the amino acid sequence of SEQ ID NO: 100 and the amino acid sequence of a light chain variable region represented by the amino acid sequence of SEQ ID NO: 101.

The monoclonal antibody against CAPRIN-1 disclosed in WO 2013/018894, wherein the antibody comprises the amino acid sequence of a heavy chain variable region represented by the amino acid sequence of SEQ ID NO: 102 and the amino acid sequence of a light chain variable region represented by the amino acid sequence of SEQ ID NO: 103, or the antibody comprises the amino acid sequence of a heavy chain variable region represented by the amino acid sequence of SEQ ID NO: 104 and the amino acid sequence of a light chain variable region represented by the amino acid sequence of SEQ ID NO: 105.

The monoclonal antibody against CAPRIN-1 disclosed in WO 2013/018892, wherein the antibody comprises the amino acid sequence of a heavy chain variable region represented by the amino acid sequence of SEQ ID NO: 106 and the amino acid sequence of a light chain variable region represented by the amino acid sequence of SEQ ID NO: 107.

The monoclonal antibody against CAPRIN-1 disclosed in WO 2013/018891, wherein the antibody comprises the amino acid sequence of a heavy chain variable region represented by the amino acid sequence of SEQ ID NO: 108 and the amino acid sequence of a light chain variable region represented by the amino acid sequence of SEQ ID NO: 109.

The monoclonal antibody against CAPRIN-1 disclosed in WO 2013/018889, wherein the antibody comprises the amino acid sequence of a heavy chain variable region represented by the amino acid sequence of SEQ ID NO: 110 and the amino acid sequence of a light chain variable region represented by the amino acid sequence of SEQ ID NO: 111.

The monoclonal antibody against CAPRIN-1 disclosed in WO 2013/018883, wherein the antibody comprises the amino acid sequence of a heavy chain variable region represented by the amino acid sequence of SEQ ID NO: 112 and the amino acid sequence of a light chain variable region represented by the amino acid sequence of SEQ ID NO: 113.

The monoclonal antibody against CAPRIN-1 disclosed in WO 2013/125636, wherein the antibody comprises the amino acid sequence of a heavy chain variable region represented by the amino acid sequence of SEQ ID NO: 114 and the amino acid sequence of a light chain variable region represented by the amino acid sequence of SEQ ID NO: 115.

The monoclonal antibody against CAPRIN-1 disclosed in WO 2013/125654, wherein the antibody comprises the amino acid sequence of a heavy chain variable region represented by the amino acid sequence of SEQ ID NO: 116 and the amino acid sequence of a light chain variable region represented by the amino acid sequence of SEQ ID NO: 117, or the antibody comprises the amino acid sequence of a heavy chain variable region represented by the amino acid sequence of SEQ ID NO: 118 and the amino acid sequence of a light chain variable region represented by the amino acid sequence of SEQ ID NO: 119.

The monoclonal antibody against CAPRIN-1 disclosed in WO 2013/125630, wherein the antibody comprises the amino acid sequence of a heavy chain variable region represented by the amino acid sequence of SEQ ID NO: 120 and the amino acid sequence of a light chain variable region represented by the amino acid sequence of SEQ ID NO: 121.

The monoclonal antibody against CAPRIN-1 disclosed in WO 2015/020212, wherein the antibody comprises the amino acid sequence of a heavy chain variable region represented by the amino acid sequence of SEQ ID NO: 122 and the amino acid sequence of a light chain variable region represented by the amino acid sequence of SEQ ID NO: 123; the antibody comprises the amino acid sequence of a heavy chain variable region represented by the amino acid sequence of SEQ ID NO: 124 and the amino acid sequence of a light chain variable region represented by the amino acid sequence of SEQ ID NO: 125; the antibody comprises the amino acid sequence of a heavy chain variable region represented by the amino acid sequence of SEQ ID NO: 126 and the amino acid sequence of a light chain variable region represented by the amino acid sequence of SEQ ID NO: 127; the antibody comprises the amino acid sequence of a heavy chain variable region represented by the amino acid sequence of SEQ ID NO: 128 and the amino acid sequence of a light chain variable region represented by the amino acid sequence of SEQ ID NO: 129; the antibody comprises the amino acid sequence of a heavy chain variable region represented by the amino acid sequence of SEQ ID NO: 130 and the amino acid sequence of a light chain variable region represented by the amino acid sequence of SEQ ID NO: 131; or the antibody comprises the amino acid sequence of a heavy chain variable region represented by the amino acid sequence of SEQ ID NO: 132 and the amino acid sequence of a light chain variable region represented by the amino acid sequence of SEQ ID NO: 133.

A nucleotide sequence was designed to express a heavy chain variable region comprising CDR1, CDR2, and CDR3 consisting of the amino acid sequences of SEQ ID NO: 36, SEQ ID NO: 37, and SEQ ID NO: 38, respectively, which are from one of the above-mentioned anti-CAPRIN-1 monoclonal antibodies, and framework region sequences of a human antibody. The nucleotide sequence was inserted into a mammalian expression vector with an insert encoding a heavy chain constant region of human IgG1. Similarly, a nucleotide sequence was designed to express a light chain variable region comprising CDR1, CDR2, and CDR3 consisting of the amino acid sequences of SEQ ID NO: 40, SEQ ID NO: 41, and SEQ ID NO: 42, respectively, and framework region sequences of a human antibody. The nucleotide sequence was inserted into a mammalian expression vector with an insert encoding a light chain constant region of human IgG1. These two recombinant expression vectors were transferred to mammalian cells according to an ordinary method, and a culture supernatant containing humanized monoclonal antibody #1 (humanized antibody #1) against CAPRIN-1 comprising CDR1, CDR2, and CDR3 of a heavy chain variable region consisting of the amino acid sequences of SEQ ID NO: 36, SEQ ID NO: 37, and SEQ ID NO: 38, respectively, and CDR1, CDR2, and CDR3 of a light chain variable region consisting of the amino acid sequences of SEQ ID NO: 40, SEQ ID NO: 41, and SEQ ID NO: 42, respectively, was obtained from the cells.

Similarly, a nucleotide sequence was designed to express a heavy chain variable region represented by SEQ ID NO: 47 comprising CDR1, CDR2, and CDR3 consisting of the amino acid sequences of SEQ ID NO: 44, SEQ ID NO: 45, and SEQ ID NO: 46, respectively, and framework region sequences of a human antibody. The nucleotide sequence was inserted into a mammalian expression vector with an insert encoding a heavy chain constant region of human IgG1. Similarly, a nucleotide sequence was designed to express a light chain variable region represented by SEQ ID NO: 51 comprising CDR1, CDR2, and CDR3 consisting of the amino acid sequences of SEQ ID NO: 48, SEQ ID NO: 49, and SEQ ID NO: 50, respectively, and framework region sequences of a human antibody. The nucleotide sequence was inserted into a mammalian expression vector with an insert encoding a heavy chain constant region of human IgG1. These two recombinant expression vectors were transferred to mammalian cells according to an ordinary method, and a culture supernatant containing humanized anti-CAPRIN-1 monoclonal antibody #2 (humanized antibody #2) comprising CDR1, CDR2, and CDR3 of a heavy chain variable region consisting of the amino acid sequences of SEQ ID NO: 44, SEQ ID NO: 45, and SEQ ID NO: 46, respectively, and CDR1, CDR2, and CDR3 of a light chain variable region consisting of the amino acid sequences of SEQ ID NO: 48, SEQ ID NO: 49, and SEQ ID NO: 50, respectively, was obtained from the cells.

A culture supernatant containing humanized anti-CAPRIN-1 monoclonal antibody #3 (humanized antibody #3) comprising CDR1, CDR2, and CDR3 of a heavy chain variable region consisting of the amino acid sequences of SEQ ID NO: 52, SEQ ID NO: 53, and SEQ ID NO: 54, respectively, and CDR1, CDR2, and CDR3 of a light chain variable region consisting of the amino acid sequences of SEQ ID NO: 56, SEQ ID NO: 57, and SEQ ID NO: 58, respectively, was prepared in a similar way.

A culture supernatant containing humanized anti-CAPRIN-1 monoclonal antibody #4 (humanized antibody #4) comprising CDR1, CDR2, and CDR3 of a heavy chain variable region consisting of the amino acid sequences of SEQ ID NO: 60, SEQ ID NO: 61, and SEQ ID NO: 62, respectively, and CDR1, CDR2, and CDR3 of a light chain variable region consisting of the amino acid sequences of SEQ ID NO: 64, SEQ ID NO: 65, and SEQ ID NO: 66, respectively, was prepared in a similar way.

Culture supernatants containing the following humanized anti-CAPRIN-1 monoclonal antibodies #9 to #41 (humanized antibodies #9 to #41) were prepared in a similar way.

Humanized monoclonal antibody #9 (humanized antibody #9) comprising the amino acid sequence of a heavy chain variable region represented by the amino acid sequence of SEQ ID NO: 68 and the amino acid sequence of a light chain variable region represented by the amino acid sequence of SEQ ID NO: 69.

Humanized monoclonal antibody #10 (humanized antibody #10) comprising the amino acid sequence of a heavy chain variable region represented by the amino acid sequence of SEQ ID NO: 70 and the amino acid sequence of a light chain variable region represented by the amino acid sequence of SEQ ID NO: 71.

Humanized monoclonal antibody #11 (humanized antibody #11) comprising the amino acid sequence of a heavy chain variable region represented by the amino acid sequence of SEQ ID NO: 72 and the amino acid sequence of a light chain variable region represented by the amino acid sequence of SEQ ID NO: 73.

Humanized monoclonal antibody #12 (humanized antibody #12) comprising the amino acid sequence of a heavy chain variable region represented by the amino acid sequence of SEQ ID NO: 74 and the amino acid sequence of a light chain variable region represented by the amino acid sequence of SEQ ID NO: 75.

Humanized monoclonal antibody #13 (humanized antibody #13) comprising the amino acid sequence of a heavy chain variable region represented by the amino acid sequence of SEQ ID NO: 76 and the amino acid sequence of a light chain variable region represented by the amino acid sequence of SEQ ID NO: 77.

Humanized monoclonal antibody #14 (humanized antibody #14) comprising the amino acid sequence of a heavy chain variable region represented by the amino acid sequence of SEQ ID NO: 78 and the amino acid sequence of a light chain variable region represented by the amino acid sequence of SEQ ID NO: 79.

Humanized monoclonal antibody #15 (humanized antibody #15) comprising the amino acid sequence of a heavy chain variable region represented by the amino acid sequence of SEQ ID NO: 80 and the amino acid sequence of a light chain variable region represented by the amino acid sequence of SEQ ID NO: 81.

Humanized monoclonal antibody #16 (humanized antibody #16) comprising the amino acid sequence of a heavy chain variable region represented by the amino acid sequence of SEQ ID NO: 82 and the amino acid sequence of a light chain variable region represented by the amino acid sequence of SEQ ID NO: 83.

Humanized monoclonal antibody #17 (humanized antibody #17) comprising the amino acid sequence of a heavy chain variable region represented by the amino acid sequence of SEQ ID NO: 84 and the amino acid sequence of a light chain variable region represented by the amino acid sequence of SEQ ID NO: 85.

Humanized monoclonal antibody #18 (humanized antibody #18) comprising the amino acid sequence of a heavy chain variable region represented by the amino acid sequence of SEQ ID NO: 86 and the amino acid sequence of a light chain variable region represented by the amino acid sequence of SEQ ID NO: 87.

Humanized monoclonal antibody #19 (humanized antibody #19) comprising the amino acid sequence of a heavy chain variable region represented by the amino acid sequence of SEQ ID NO: 88 and the amino acid sequence of a light chain variable region represented by the amino acid sequence of SEQ ID NO: 89.

Humanized monoclonal antibody #20 (humanized antibody #20) comprising the amino acid sequence of a heavy chain variable region represented by the amino acid sequence of SEQ ID NO: 90 and the amino acid sequence of a light chain variable region represented by the amino acid sequence of SEQ ID NO: 91.

Humanized monoclonal antibody #21 (humanized antibody #21) comprising the amino acid sequence of a heavy chain variable region represented by the amino acid sequence of SEQ ID NO: 92 and the amino acid sequence of a light chain variable region represented by the amino acid sequence of SEQ ID NO: 93.

Humanized monoclonal antibody #22 (humanized antibody #22) comprising the amino acid sequence of a heavy chain variable region represented by the amino acid sequence of SEQ ID NO: 94 and the amino acid sequence of a light chain variable region represented by the amino acid sequence of SEQ ID NO: 95.

Humanized monoclonal antibody #23 (humanized antibody #23) comprising the amino acid sequence of a heavy chain variable region represented by the amino acid sequence of SEQ ID NO: 96 and the amino acid sequence of a light chain variable region represented by the amino acid sequence of SEQ ID NO: 97.

Humanized monoclonal antibody #24 (humanized antibody #24) comprising the amino acid sequence of a heavy chain variable region represented by the amino acid sequence of SEQ ID NO: 98 and the amino acid sequence of a light chain variable region represented by the amino acid sequence of SEQ ID NO: 99.

Humanized monoclonal antibody #25 (humanized antibody #25) comprising the amino acid sequence of a heavy chain variable region represented by the amino acid sequence of SEQ ID NO: 100 and the amino acid sequence of a light chain variable region represented by the amino acid sequence of SEQ ID NO: 101.

Humanized monoclonal antibody #26 (humanized antibody #26) comprising the amino acid sequence of a heavy chain variable region represented by the amino acid sequence of SEQ ID NO: 102 and the amino acid sequence of a light chain variable region represented by the amino acid sequence of SEQ ID NO: 103.

Humanized monoclonal antibody #27 (humanized antibody #27) comprising the amino acid sequence of a heavy chain variable region represented by the amino acid sequence of SEQ ID NO: 104 and the amino acid sequence of a light chain variable region represented by the amino acid sequence of SEQ ID NO: 105.

Humanized monoclonal antibody #28 (humanized antibody #28) comprising the amino acid sequence of a heavy chain variable region represented by the amino acid sequence of SEQ ID NO: 106 and the amino acid sequence of a light chain variable region represented by the amino acid sequence of SEQ ID NO: 107.

Humanized monoclonal antibody #29 (humanized antibody #29) comprising the amino acid sequence of a heavy chain variable region represented by the amino acid sequence of SEQ ID NO: 108 and the amino acid sequence of a light chain variable region represented by the amino acid sequence of SEQ ID NO: 109.

Humanized monoclonal antibody #30 (humanized antibody #30) comprising the amino acid sequence of a heavy chain variable region represented by the amino acid sequence of SEQ ID NO: 110 and the amino acid sequence of a light chain variable region represented by the amino acid sequence of SEQ ID NO: 111.

Humanized monoclonal antibody #31 (humanized antibody #31) comprising the amino acid sequence of a heavy chain variable region represented by the amino acid sequence of SEQ ID NO: 112 and the amino acid sequence of a light chain variable region represented by the amino acid sequence of SEQ ID NO: 113.

Humanized monoclonal antibody #32 (humanized antibody #32) comprising the amino acid sequence of a heavy chain variable region represented by the amino acid sequence of SEQ ID NO: 114 and the amino acid sequence of a light chain variable region represented by the amino acid sequence of SEQ ID NO: 115.

Humanized monoclonal antibody #33 (humanized antibody #33) comprising the amino acid sequence of a heavy chain variable region represented by the amino acid sequence of SEQ ID NO: 116 and the amino acid sequence of a light chain variable region represented by the amino acid sequence of SEQ ID NO: 117.

Humanized monoclonal antibody #34 (humanized antibody #34) comprising the amino acid sequence of a heavy chain variable region represented by the amino acid sequence of SEQ ID NO: 118 and the amino acid sequence of a light chain variable region represented by the amino acid sequence of SEQ ID NO: 119.

Humanized monoclonal antibody #35 (humanized antibody #35) comprising the amino acid sequence of a heavy chain variable region represented by the amino acid sequence of SEQ ID NO: 120 and the amino acid sequence of a light chain variable region represented by the amino acid sequence of SEQ ID NO: 121.

Humanized monoclonal antibody #36 (humanized antibody #36) comprising the amino acid sequence of a heavy chain variable region represented by the amino acid sequence of SEQ ID NO: 122 and the amino acid sequence of a light chain variable region represented by the amino acid sequence of SEQ ID NO: 123.

Humanized monoclonal antibody #37 (humanized antibody #37) comprising the amino acid sequence of a heavy chain variable region represented by the amino acid sequence of SEQ ID NO: 124 and the amino acid sequence of a light chain variable region represented by the amino acid sequence of SEQ ID NO: 125.

Humanized monoclonal antibody #38 (humanized antibody #38) comprising the amino acid sequence of a heavy chain variable region represented by the amino acid sequence of SEQ ID NO: 126 and the amino acid sequence of a light chain variable region represented by the amino acid sequence of SEQ ID NO: 127.

Humanized monoclonal antibody #39 (humanized antibody #39) comprising the amino acid sequence of a heavy chain variable region represented by the amino acid sequence of SEQ ID NO: 128 and the amino acid sequence of a light chain variable region represented by the amino acid sequence of SEQ ID NO: 129.

Humanized monoclonal antibody #40 (humanized antibody #40) comprising the amino acid sequence of a heavy chain variable region represented by the amino acid sequence of SEQ ID NO: 130 and the amino acid sequence of a light chain variable region represented by the amino acid sequence of SEQ ID NO: 131.

Humanized monoclonal antibody #41 (humanized antibody #41) comprising the amino acid sequence of a heavy chain variable region represented by the amino acid sequence of SEQ ID NO: 132 and the amino acid sequence of a light chain variable region represented by the amino acid sequence of SEQ ID NO: 133.

On the basis of humanized antibody #1 among these anti-CAPRIN-1 monoclonal antibodies, a nucleotide sequence was designed to express a heavy chain variable region heavy chain variable region comprising CDR1, CDR2, and CDR3 consisting of the amino acid sequences of SEQ ID NO: 36, SEQ ID NO: 37, and SEQ ID NO: 38, respectively, and framework region sequences of a human antibody. The nucleotide sequence was inserted into a mammalian expression vector with an insert encoding a heavy chain constant region of human IgG1 in which serine (Ser) at amino acid position 239 in EU numbering is substituted with aspartic acid (Asp), and isoleucine (Ile) at amino acid position 332 in EU numbering is substituted with glutamic acid (Glu). Also, a nucleotide sequence was designed to express the amino acid sequence of a light chain variable region comprising CDR1, CDR2, and CDR3 consisting of the amino acid sequences of SEQ ID NO: 40, SEQ ID NO: 41, and SEQ ID NO: 42, respectively, and framework region sequences of a human antibody. The nucleotide sequence was inserted into a mammalian expression vector with an insert encoding a light chain constant region of human IgG1. These two recombinant expression vectors were transferred to mammalian cells according to an ordinary method; and a culture supernatant containing humanized monoclonal antibody #5 (humanized antibody #5) against CAPRIN-1 composed of the full-length heavy chain amino acid sequence consisting of the heavy chain variable region designed above and the heavy chain constant region of human IgG1 in which serine (Ser) at amino acid position 239 in EU numbering is substituted with aspartic acid (Asp), and isoleucine (Ile) at amino acid position 332 in EU numbering is substituted with glutamic acid (Glu), and the full-length light chain amino acid sequence consisting of the light chain variable region designed above and the human light chain constant region, was obtained from the cells.

A culture supernatant containing humanized anti-CAPRIN-1 monoclonal antibody #6 (humanized antibody #6) comprising the amino acid sequence of the heavy chain variable region and the amino acid sequence of the light chain variable region of the humanized antibody #2 produced above was prepared in a similar way.

A culture supernatant containing humanized anti-CAPRIN-1 monoclonal antibody #7 (humanized antibody #7) comprising the amino acid sequence of the heavy chain variable region and the amino acid sequence of the light chain variable region of the humanized antibody #3 produced above was prepared in a similar way.

A culture supernatant containing humanized anti-CAPRIN-1 monoclonal antibody #8 (humanized antibody #8) comprising the amino acid sequence of the heavy chain variable region and the amino acid sequence of the light chain variable region of the humanized antibody #4 produced above was prepared in a similar way.

Culture supernatants containing each of humanized anti-CAPRIN-1 antibodies #42 to #74 (humanized antibodies #42 to #74) comprising the amino acid sequence of the heavy chain variable region and the amino acid sequence of the light chain variable region of each of the humanized antibodies #9 to #41 produced above were prepared in a similar way.

The obtained culture supernatants containing each of the humanized anti-CAPRIN-1 monoclonal antibodies #1 to #74 were subjected to purification using Hitrap Protein A Sepharose FF (GE Healthcare) according to an ordinary method. The buffer was replaced with PBS(-). The resultant was filtered through a 0.22 µm filter (Merck Millipore Corp.) to prepare the humanized antibodies.

### (Example 3) Preparation of conjugate of anti-CAPRIN-1 antibody and PBD derivative (SGD-1910)

Conjugates of anti-CAPRIN-1 polyclonal antibodies #1 to #6 described in Example 1 with SGD-1910 (CAS#1342820-51-2) were prepared according to an ordinary method. The purified anti-CAPRIN-1 polyclonal antibody #1 was dissolved in PBS(-) at 10 mg/ml. To the solution containing the polyclonal antibody #1, a PBS(-) solution containing 100 mM EDTA and 50 mM L-cysteine was added in an amount one tenth that of the antibody solution, and the resultant was then subjected to a reduction reaction at 4°C for 1 hour. After the reaction, the solution was subj ected to buffer replacement with a solution of PBS(-) containing 1 mM EDTA with the use of the Zeba^{(™)}Spin Desalting Column (MWCO: 70 k or 40 k), and the resultant was left to stand at room temperature for 18 to 72 hours to reoxidate the polyclonal antibody. Propylene glycol was added to 50% in the reoxidated polyclonal antibody #1. A solution of 10 mM SGD-1910 in DMSO was added to the solution containing the polyclonal antibody #1, so as to bring the molar ratio of the antibody to SDG-1910 to 1:5, and a reaction was allowed to proceed at room temperature for 1 hour or longer. After the reaction, SGD-1910 that did not bind to the antibody was removed with the use of the Zeba^{(™)}Spin Desalting Column (MWCO: 70 k or 40 k), and the solvent was replaced with PBS(-) (replacement was performed 2 times). After the buffer was replaced with PBS(-), the resultant was filtered through a 0.22-µm sterilization filter, and a solution containing the anti-CAPRIN-1 polyclonal antibody #1-SDG-1910 of the present invention (Conjugate 1) was thus obtained. Solutions containing conjugates of each of the anti-CAPRIN-1 polyclonal antibodies #2 to #6 and SDG-1910 were also obtained (Conjugate 2: the conjugate of the anti-CAPRIN-1 polyclonal antibody #2; Conjugate 3: the conjugate of the anti-CAPRIN-1 polyclonal antibody #3; Conjugate 4: the conjugate of the anti-CAPRIN-1 polyclonal antibody #4; Conjugate 5: the conjugate of the anti-CAPRIN-1 polyclonal antibody #5; Conjugate 6: the conjugate of the anti-CAPRIN-1 polyclonal antibody #6). In the same manner as described above, a solution containing the conjugate of the anti-CAPRIN-1 monoclonal antibody described in Example 2 (the humanized antibody #4) and SDG-1910 (Conjugate 10) was obtained. In the same manner as described above, a solution containing the conjugate of the rabbit control antibody unreactive with the CAPRIN-1 protein described in Example 1 and the SDG-1910 linker (Control conjugate 1) was obtained in the same manner.

In addition, a conjugate of the anti-CAPRIN-1 monoclonal antibody described in Example 2 (humanized antibody #1) and SGD-1910 (CAS#1342820-51-2) was prepared according to an ordinary method. The purified humanized antibody #1 was dissolved in PBS(-) at 10 mg/ml. To the solution containing the humanized antibody #1, a PBS(-) solution containing TCEP (final concentration: 10 mM) was added, and the resultant was then subjected to a reduction reaction at room temperature for 1 hour. After the reaction, the solution was subjected to buffer replacement with a solution of PBS(-) containing 1 mM EDTA with the use of the Zeba^{(™)}Spin Desalting Column (MWCO: 70 k or 40 k), and an equivalent amount of propylene glycol was added thereto. A solution of 10 mM SGD-1910 in DMSO was added to the solution containing the humanized antibody #1, so as to bring the molar ratio of the antibody to SDG-1910 to 1:3, and a reaction was allowed to proceed at room temperature for 1 hour or longer. After the reaction, SGD-1910 that did not bind to the antibody was removed with the use of the Zeba^{(™)}Spin Desalting Column (MWCO: 70 k or 40 k), and the solvent was replaced with PBS(-) (replacement was performed 2 times). After the buffer was replaced with PBS(-), the resultant was filtered through a 0.22-µm sterilization filter, and a solution containing the humanized antibody #1-SDG-1910 of the present invention (Conjugate 7) was thus obtained. Solutions containing conjugates of each of the anti-CAPRIN-1 polyclonal antibodies #2 to #6 and the humanized antibodies #2, #3, and #5 to #74 and SDG-1910 were also obtained (Conjugate 8, Conjugate 9, Conjugate 11 to Conjugate 53). In the same manner as described above, a solution containing the conjugate of the human IgG control antibody unreactive with the CAPRIN-1 protein and the SDG-1910 linker (Control conjugate 2) was obtained in a similar way.

### (Example 4) Specific reactivity of conjugate with CAPRIN-1 protein and CAPRIN-1-expressing cancer cell

Conjugates prepared in Example 3 (the anti-CAPRIN-1 antibody-SDG-1910 conjugates) were assayed for their specific reactivity with a CAPRIN-1 protein and their reactivity with the cell membrane surface of human cancer cells and mouse cancer cells expressing a CAPRIN-1 protein.

The specific reactivity with the CAPRIN-1 protein was determined by ELISA. A 1 µg/mL CAPRIN-1 protein solution was added at 100 µL/well to a 96-well plate, and the plate was left to stand at 4°C for 18 hours. Each well was washed with PBS-T three times. Then, a 0.5% bovine serum albumin (BSA) solution was added at 400 µL/well, and the plate was left to stand at room temperature for 3 hours. The solution was removed, and the wells were washed with 400 µL/well of PBS-T three times. Then, each of the solutions containing Conjugates 1 to 6 and Control conjugate 1 was added at 100 µL/well, and the plate was left to stand at room temperature for 2 hours. Each well was washed with PBS-T three times. Then, an HRP-labeled anti-rabbit IgG antibody diluted 5000-fold with PBS was added at 100 µL/well, and left to stand at room temperature for 1 hour. Each well was washed with PBS-T three times. Then, a TMB substrate solution was added at 100 µL/well, and left to stand for 15 to 30 minutes for chromogenic reaction. After the color development, the reaction was terminated by the addition of 1 N sulfuric acid at 100 µL/well, and the absorbance values at 450 nm and 595 nm were measured using an absorption spectrometer. As a result, Conjugates 1 to 6 were found to exhibit higher absorbance values than that of Control conjugate 1 as a negative control and to exhibit specific reactivity with the CAPRIN-1 protein.

The specific reactivity of each of the anti-CAPRIN-1 antibody-SDG-1910 conjugates prepared with the use of the humanized antibodies #1 to #74 in Example 3 with the CAPRIN-1 protein was detected by ELISA using the HRP-labeled anti-human antibody. As a result, the anti-CAPRIN-1 antibody-SDG-1910 conjugates using the humanized antibodies #1 to #74 were found to exhibit higher absorbance values than that of human IgG (SIGMA) and Control conjugate 2 as negative controls and to exhibit specific reactivity with the CAPRIN-1 protein.

Next, the reactivity with the cell membrane surface of CAPRIN-1-expressing cancer cells was verified by flow cytometry. Human breast cancer cells BT-474 (from ATCC) or mouse breast cancer cells 4T1 (from ATCC) were centrifuged in 1.5 mL microcentrifuge tubes (2 x 10⁵ cells per tube). 100 µL of the solutions containing each of Conjugates 1 to 6 and Control conjugate 1 was then each added to separate tubes. The tubes were left to stand at 4°C for 1 hour. After washing with PBS, Alexa 488-labeled anti-rabbit IgG (H + L) diluted 100-fold with PBS(-) containing 0.5% FBS (0.5% FBS-PBS(-)) was added thereto, and the tubes were left to stand at 4°C for 1 hour. After washing with 0.5% FBS-PBS(-), the cells were allowed to react with BD Horizon Fixable Viability Stain (FVS) Reagents (FVS450, Becton, Dickinson and Company) to dye the dead cells, and the fluorescence intensity was measured using FACSFortessa^{™} (Becton, Dickinson and Company). As a result, the anti-CAPRIN-1 antibody-SDG-1910 conjugates (Conjugates 1 to 6) were found to exhibit a higher fluorescence intensity than that of Control conjugate 1 as a negative control, i.e., to strongly react with the cell surface of the human cancer cells BT474 expressing CAPRIN-1.

Also, the specific reactivity of each of the anti-CAPRIN-1 antibody-SDG-1910 conjugates prepared with the use of the humanized antibodies #1 to #74 with the human cancer cells BT474 and the mouse cancer cells 4T1 expressing CAPRIN-1 was detected using the Alexa488-labeled anti-human IgG (H + L) antibody. As a result, the anti-CAPRIN-1 antibody-SDG-1910 conjugates prepared with the use of the humanized antibodies #1 to #74 were found to exhibit a higher fluorescence intensity than that of Control conjugate 2 as a negative control, i.e., to strongly react with the cell surface of the human cancer cells BT474 and the mouse cancer cells 4T1 expressing CAPRIN-1.

The reactivity of each of the anti-CAPRIN-1 antibody-SDG-1910 conjugates prepared with the use of the anti-CAPRIN-1 polyclonal antibodies and the humanized antibodies #1 to #74 in Example 3 with various human cancer cells and mouse cancer cells was verified in a similar way. The anti-CAPRIN-1 antibody-SDG-1910 conjugates prepared in Example 3 were found to exhibit stronger fluorescence intensities for human cancer cells that have been verified to express the CAPRIN-1 gene, and that have been verified to express CAPRIN-1 on the cell membrane surface thereof; i.e., breast cancer cells (BT-474), colorectal cancer cells (HT-29, HCT116, Lovo), lung cancer cells (A549), stomach cancer cells (NCI-N87), uterine cancer cells (HEC-1-A), prostate cancer cells (22Rv1), pancreatic cancer cells (Panc10.5), liver cancer cells (HepG2), ovary cancer cells (MCAS, SKOV3, TOV112D), kidney cancer cells (Caki-2), brain tumor cells (U-87MG), bladder cancer cells (T24, UMUC3), bile duct cancer cells (KKU213), fibrosarcoma cells (HT-1080), esophagus cancer cells (OE33), leukemia cells (OCI-AML5), lymphoma cells (Ramos), gallbladder cancer cells (TGBC14TKB), melanoma cells (Malme-3M), and head and neck cancer cells (FaDu), and for mouse kidney cancer cells (Renca) and mouse breast cancer cells (4T1) that have been verified to express the CAPRIN-1 gene, than those of Control conjugate 1 and Control conjugate 2 as negative controls and to strongly react with the cell membrane surface of the cancer cells expressing CAPRIN-1.

### (Example 5) Internalization

Internalization of each of the anti-CAPRIN-1 antibody-SDG-1910 conjugates prepared with the use of the anti-CAPRIN-1 polyclonal antibodies and the humanized antibodies #1 to #74 in Example 3 in cancer cells was evaluated *in vitro.* The solution of the anti-CAPRIN-1 antibody-SDG-1910 conjugate (4 µg/mL) was mixed with the equivalent amount of the solution of Incucyte^{(™)}Human Fabfluor-pH Antibody Labeling Dye (2 µg/mL), and the mixture was left to stand at 37°C for 1 hour to prepare a composite of the anti-CAPRIN-1 antibody-SDG-1910 conjugate and Human Fabfluor-pH Antibody Labeling Dye. The composite (100 µL) was added to 2 × 10⁵ cancer cells HT29, and a reaction was allowed to proceed at 37°C for 6 hours. After the reaction, fluorescence intensity was measured by flow cytometry to evaluate internalization according to an ordinary method. As a result of evaluation, the anti-CAPRIN-1 antibody-SDG-1910 conjugates were found to exhibit stronger fluorescence intensity than that of a negative control (HT29 that reacted with only the anti-CAPRIN-1 antibody-SDG-1910 conjugate). The results demonstrate that the anti-CAPRIN-1 antibody-SDG-1910 conjugates are internalized in human cancer cells. Internalization of the HER2-targeting antibody, Trastuzumab, the EGFR-targeting antibody, Cetuximab, the Nectin-4-targeting ADC, Enfortumab vedotin, and the TROP2-targeting ADC, Sacituzumab govitecan, in HT29 was also examined by flow cytometry. As a result, the anti-CAPRIN-1 antibody-SDG-1910 conjugates were found to exhibit higher fluorescence intensity than that of such antibodies.

### (Example 6) Antitumor effect of conjugate

The antitumor activity of each of the anti-CAPRIN-1 antibody-SDG-1910 conjugates prepared with the use of the anti-CAPRIN-1 polyclonal antibodies and the humanized antibodies #1 to #74 in Example 3 on cancer cells was evaluated *in vitro.* The antitumor activity thereof on the human cancer cells that have been verified to express CAPRIN-1 on the cell membrane surface thereof in Example 4; i.e., breast cancer cells (BT-474), colorectal cancer cells (HT-29, HCT116), stomach cancer cells (NCI-N87), uterine cancer cells (Ishikawa), pancreatic cancer cells (Panc10.5), ovary cancer cells (MCAS, TOV112D), and head and neck cancer cells (FaDu), was evaluated.

A solution of the cancer cells cultured by a standard technique was applied to a black 96-well plate capable of performing cell culture (96 well optical Btm Plt Polymer Base Black w/Lid Cell Culture Sterile PS 165305, Thermo) at 90 µL/well to bring the cell density to 1 to 4 × 10⁴ cells/well, and culture was then performed in an incubator in the presence of 5% CO₂ at 37°C for 18 to 24 hours. Thereafter, solutions of each of the anti-CAPRIN-1 antibody-SDG-1910 conjugates prepared with the use of the anti-CAPRIN-1 polyclonal antibodies and the humanized antibodies #1 to #74 in Example 3 were added at 10 µL/well to adjust the antibody density to 10⁻² µg/mL to 10² µg/mL, and incubation was then performed in an incubator in the presence of 5% CO₂ at 37°C. The cell survival rates 3 to 4 days after the start of the incubation were determined by the Cell Titer Glow Luminescent Cell Viability Assay (#7573, Promega). A substrate was added at 100 µL/well, the plate was shaken using a plate shaker for 2 minutes, the plate was left to stand for 10 minutes, and the luminescence signal intensity was determined using SpectraMax^{®} iD3 (MOLECULAR DEVICE) to calculate the ATP content of the survived cells.

As a result, the rate of cancer cells survived in wells containing the anti-CAPRIN-1 antibody-SDG-1910 conjugates of the anti-CAPRIN-1 polyclonal antibodies and the humanized antibodies #1 to #74 of the present invention at 1 µg/mL or higher was found to be 70% or lower, relative to the rate of cancer cells survived in wells not containing the conjugate (100%). The results demonstrate that a conjugate of an antibody against CAPRIN-1 and PBD exhibits antitumor activity on cancer cells.

### (Example 7) Antitumor effects of conjugates on cancer-bearing mice

The anti-CAPRIN-1 antibody-SDG-1910 conjugates prepared in Example 3 (Conjugate 7 and Conjugate 10) were evaluated for their *in vivo* antitumor effects on cancer-bearing mice. The conjugates of the present invention were examined for their antitumor effects using NOD-SCID mice in which human-derived cancer cells expressing the CAPRIN-1 protein were transplanted. Human colorectal cancer cells HT29 were mixed with Matrigel (Sigma) and subcutaneously transplanted at 10⁷ cells/mouse to the mice, which were then grown until tumor became 50 mm³ or larger to prepare cancer-bearing mice. HT29 expresses the CAPRIN-1 protein on the cell membrane surface. As shown in Example 4, Conjugates 7 and 10 each bind to HT29. Conjugates 7 and 10 were each administered at 0.3 mg/kg to the tail veins of HT29-bearing mice. The administration was carried out once a week a total of two times. A solution containing a conjugate of Trastuzumab (Chugai Pharmaceutical Co., Ltd.) and SDG-1910 was prepared by the method described in Example 3, so that a drug equivalent to the drug binding to the conjugate of an antibody against CAPRIN-1 would bind thereto, and the resultant was administered as a comparative control in the same amount as above to the cancer-bearing mice. HT29 expresses HER2 protein, which is a target antigen of Trastuzumab, on the cell membrane surface. The conjugate of Trastuzumab and PBD specifically binds to HT29. PBS(-) was administered to the cancer-bearing mice, for a negative control.

The tumor sizes of the cancer-bearing mice after the administration were measured using calipers over time, and tumor volumes were calculated according to an ordinary method based on the expression: (Length of the major axis of tumor) x (Length of the minor axis of tumor)² x 0.5. As a result, the mice given Conjugates 7 and 10 had less than 50% tumor volume 7 days after the start of the administration relative to the tumor volume of the negative control (100%). All the cancer-bearing mice given Conjugates 7 and 10 had less than 50% tumor volumes relative to the tumor volumes of the cancer-bearing mice given only the antibody against CAPRIN-1 used for the conjugates indicated above (100%). In contrast, the mice given a solution containing a control conjugate of Trastuzumab and PBD had 80% or more tumor volumes relative to the tumor volumes of the negative controls. In addition, the cancer-bearing mice given the conjugate were less than 75% relative to the tumor volumes of the cancer-bearing mice given only Trastuzumab (100%).

The results of evaluation demonstrate that the conjugate of an antibody against CAPRIN-1 and SDG-1910 prepared in Example 3 exhibits significantly higher antitumor effects than those of negative controls (a no-treatment control group). The results also demonstrate that the conjugate of interest exhibits significantly higher antitumor effects than those of control conjugates of Trastuzumab and SDG-1910.

### (Example 8) Preparation of conjugate of anti-CAPRIN-1 antibody and PBD derivative (Tesirine)

In the same manner as in Example 3, according to an ordinary method, SATA was bound as a spacer to the lysine residue of the anti-CAPRIN-1 polyclonal antibody #1 described in Example 1, and a PBD derivative; i.e., Tesirine represented by the chemical formula indicated below, was bound to the antibody through the maleimide group to obtain a solution containing the conjugate (Conjugate 54). In the same manner, a conjugate of the anti-CAPRIN-1 monoclonal antibody described in Example 2; i.e., the humanized antibody #1, and Tesirine was prepared to obtain a solution containing the conjugate (Conjugate 60). In the same manner, conjugates of the anti-CAPRIN-1 polyclonal antibodies #2 to #6 and the anti-CAPRIN-1 monoclonal antibodies (the humanized antibodies #2 to #47) and Tesirine derivatives were prepared. As for the rabbit control antibody and the human IgG control antibody unreactive with the CAPRIN-1 protein described in Example 1, solutions each containing a conjugate of the antibody and Tesirine were also prepared in the same manner as described above (Control conjugate 3, Control conjugate 4). The conjugates thus prepared were evaluated in the same manner as described in Examples 4 to 6. As a result, the conjugates were found to exhibit specific reactivity with the CAPRIN-1 protein and CAPRIN-1-expressing cancer cells, internalization therein, and antitumor activity thereon.

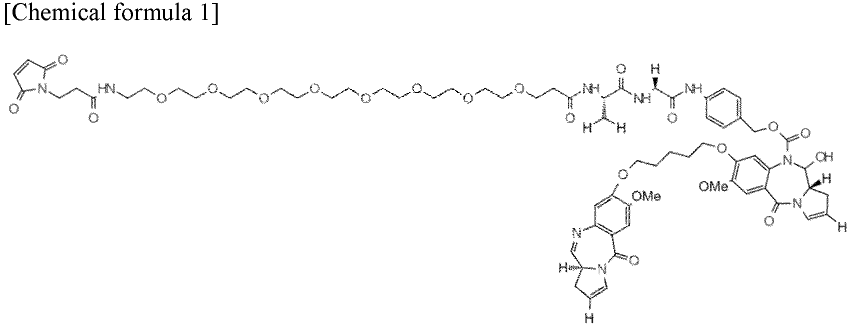

### (Example 9) Antitumor effect of conjugate of anti-CAPRIN-1 antibody and PBD derivative (Tesirine) on cancer-bearing mice

Conjugates 60 and 63 prepared in Example 8 were evaluated for their *in vivo* antitumor effects on cancer-bearing mice. The conjugates of the present invention were examined for their antitumor effects using NOD-SCID mice in which human-derived cancer cells expressing the CAPRIN-1 protein were transplanted. Human colorectal cancer cells HT29 were mixed with Matrigel (Sigma) and subcutaneously transplanted at 10⁷ cells/mouse to the mice, which were then grown until tumor became 100 to 200 mm³ or larger to prepare cancer-bearing mice. HT29 expresses the CAPRIN-1 protein on the cell membrane surface, and Conjugates 60 and 63 each bind to HT29. Conjugates 60 and 63 were each administered at 2 mg/kg and 6 mg/kg, respectively, to the tail veins of HT29-bearing mice. The administration was carried out once. PBS(-) was administered to the cancer-bearing mice, for a negative control.

The tumor sizes of the cancer-bearing mice after the administration were measured using calipers over time, and tumor volumes were calculated according to an ordinary method based on the expression: (Length of the major axis of tumor) x (Length of the minor axis of tumor)² x 0.5. As a result, the mice given Conjugates 60 and 63 at 2 mg/kg had less than 50% tumor volume 7 days after the start of the administration relative to the tumor volume of the negative control (100%). In contrast, the tumor volumes of the cancer-bearing mice given Conjugates 60 and 63 at 6 mg/kg were decreased.

The results of evaluation demonstrate that the conjugate of an antibody against CAPRIN-1 and Tesirine prepared in Example 8 exhibits significantly higher antitumor effects than those of negative controls (a no-treatment control group).

### (Example 10) Preparation of conjugate of anti-CAPRIN-1 antibody and IGN derivative (DGN549)

In the same manner as in Example 3, according to an ordinary method, SATA was bound as a spacer to the lysine residue of the anti-CAPRIN-1 polyclonal antibody #1 described in Example 1, and an IGN derivative; i.e., DGN549 represented by the chemical formula indicated below, was bound to the antibody through the maleimide group to obtain a solution containing the conjugate (Conjugate 107). In the same manner, a conjugate of the anti-CAPRIN-1 monoclonal antibody described in Example 2; i.e., the humanized antibody #1, and the DGN549 was prepared to obtain a solution containing the conjugate (Conjugate 113). In the same manner, conjugates of the anti-CAPRIN-1 polyclonal antibodies #2 to #6 and the anti-CAPRIN-1 monoclonal antibodies (the humanized antibodies #2 to #47) and DGN549 were prepared. As for the rabbit control antibody and the human IgG control antibody unreactive with the CAPRIN-1 protein described in Example 1, solutions each containing a conjugate of the antibody and DGN549 were obtained (Control conjugate 5, Control conjugate 6). The conjugates thus prepared were evaluated in the same manner as described in Examples 4 to 6. As a result, the conjugates were found to exhibit specific reactivity with the CAPRIN-1 protein and CAPRIN-1-expressing cancer cells, internalization therein, and antitumor activity thereon.

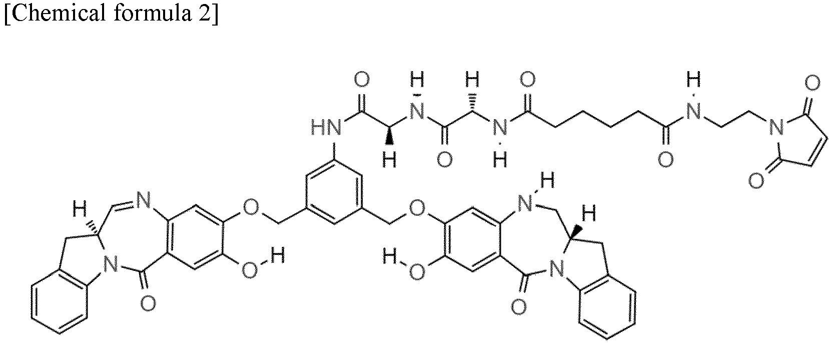

### (Example 11) Antitumor effect of conjugate of anti-CAPRIN-1 antibody and IGN (DGN549) on cancer-bearing mice -1

Conjugates 107 and 113 prepared in Example 10 were evaluated for their *in vivo* antitumor effects on cancer-bearing mice. The conjugates of the present invention were examined for their antitumor effects using NOD-SCID mice in which human-derived cancer cells expressing the CAPRIN-1 protein were transplanted. Human head and neck cancer cells FaDu were mixed with Matrigel (Sigma) and subcutaneously transplanted at 10⁷ cells/mouse to the mice, which were then grown until tumor became 100 to 200 mm³ or larger to prepare cancer-bearing mice. FaDu expresses the CAPRIN-1 protein on the cell membrane surface, and Conjugates 107 and 113 each bind to FaDu. Conjugates 107 and 113 were each administered at 2 mg/kg and 6 mg/kg, respectively, to the tail veins of FaDu-bearing mice. The administration was carried out once. PBS(-) was administered to the cancer-bearing mice, for a negative control.

The tumor sizes of the cancer-bearing mice after the administration were measured using calipers over time, and tumor volumes were calculated according to an ordinary method based on the expression: (Length of the major axis of tumor) x (Length of the minor axis of tumor)² x 0.5. As a result, the mice given Conjugates 103 and 117 at 2 mg/kg had less than 50% tumor volume 7 days after the start of the administration relative to the tumor volume of the negative control (100%). In contrast, the tumor volumes of the cancer-bearing mice given Conjugates 103 and 117 at 6 mg/kg were decreased.

The results of evaluation demonstrate that the conjugate of an antibody against CAPRIN-1 and DGN549 prepared in Example 10 exhibits significantly higher antitumor effects than those of negative controls (a no-treatment control group).

### (Example 12) Antitumor effect of conjugate of anti-CAPRIN-1 antibody and IGN (DGN549) on cancer-bearing mice -2

Conjugates 107 and 113 prepared in Example 10 were evaluated for their *in vivo* antitumor effects on cancer-bearing mice. The conjugates of the present invention were examined for their antitumor effects using C.B-17SCID mice in which human-derived cancer cells expressing the CAPRIN-1 protein were transplanted. Human ovary cancer cells TOV112D were mixed with Matrigel (Sigma) and subcutaneously transplanted at 5 × 10⁶ cells/mouse to the mice, which were then grown until tumor became 150 to 200 mm³ or larger on average to prepare cancer-bearing mice. TOV112D expresses the CAPRIN-1 protein on the cell membrane surface, and Conjugates 107 and 113 each bind to TOV112D. Conjugates 107 and 113 were each administered at 0.5 mg/kg to the tail veins of TOV112D-bearing mice. The administration was carried out once. PBS(-) was administered to the cancer-bearing mice, for a negative control.

The tumor sizes of the cancer-bearing mice after the administration were measured using calipers over time, and tumor volumes were calculated according to an ordinary method based on the expression: (Length of the major axis of tumor) x (Length of the minor axis of tumor)² x 0.5. As a result, the cancer-bearing mice given Conjugates 103 and 117 had 13% or less tumor volume 15 days after the start of the administration relative to the tumor volume of the negative control (100%).

The results of evaluation demonstrate that the conjugate of an antibody against CAPRIN-1 and DGN549 prepared in Example 10 exhibits significantly higher antitumor effects than those of negative controls (a no-treatment control group).

### (Example 13) Antitumor effect of conjugate of anti-CAPRIN-1 antibody and IGN (DGN549) on cancer-bearing mice -3

Conjugates 107 and 113 prepared in Example 10 were evaluated for their *in vivo* antitumor effects on cancer-bearing mice. The conjugates of the present invention were examined for their antitumor effects using Balb/c-nu/nu (Balb/c nude) mice in which human-derived cancer cells expressing the CAPRIN-1 protein were transplanted. Human bladder cancer cells UMUC3 were mixed with Matrigel (Sigma) and subcutaneously transplanted at 5 × 10⁶ cells/mouse to the mice, which were then grown until tumor became 120 to 150 mm³ or larger on average to prepare cancer-bearing mice. UMUC3 expresses the CAPRIN-1 protein on the cell membrane surface, and Conjugates 107 and 113 each bind to UMUC3. Conjugates 107 and 113 were each administered at 0.5 mg/kg to the tail veins of UMUC3-bearing mice. The administration was carried out once. PBS(-) was administered to the cancer-bearing mice, for a negative control.

The tumor sizes of the cancer-bearing mice after the administration were measured using calipers over time, and tumor volumes were calculated according to an ordinary method based on the expression: (Length of the major axis of tumor) x (Length of the minor axis of tumor)² x 0.5. As a result, the cancer-bearing mice given Conjugates 103 and 117 had 11% or less tumor volume 15 days after the start of the administration relative to the tumor volume of the negative control (100%).

The results of evaluation demonstrate that the conjugate of an antibody against CAPRIN-1 and DGN549 prepared in Example 10 exhibits significantly higher antitumor effects than those of negative controls (a no-treatment control group).

### (Example 14) Preparation of conjugate of anti-CAPRIN-1 antibody and PDD derivative

In the same manner as in Example 3, according to an ordinary method, SATA was bound as a spacer to the lysine residue of the anti-CAPRIN-1 polyclonal antibody #1 described in Example 1, and a compound represented by the chemical formula indicated below composed of a PDD derivative; i.e., the compound described in Example 41 of U.S. Patent No. 10975072 B2 ((S)-N-(4-aminophenyl)-4-(4-(4-(4-((2-methoxy-12-oxo-6a,7,8,9,10,12-hexahydrobenzo[e]pyrido[1,2-a][1,4]diazepin-3-yl)oxy)butanamide)-1-methyl-1H-pyrrole-2-carboxamide)phenyl)-1-methyl-1H-pyrrole-2-carboxamide) and a linker comprising a maleimide group bound thereto, was bound to the antibody through the maleimide group to obtain a solution containing the conjugate (Conjugate 160). In the same manner, a conjugate of the anti-CAPRIN-1 monoclonal antibody described in Example 2 (the humanized antibody #1) and the compound indicated above was prepared to obtain a solution containing the conjugate (Conjugate 166). In the same manner, conjugates of the anti-CAPRIN-1 polyclonal antibodies #2 to #6 and the anti-CAPRIN-1 monoclonal antibodies (the humanized antibodies #2 to #47) and the compound indicated above were prepared. As for the rabbit control antibody and the human IgG control antibody unreactive with the CAPRIN-1 protein described in Example 1, solutions each containing a conjugate of the antibody and the PDD derivative were obtained in a similar way (Control conjugate 7, Control conjugate 8). The conjugates thus prepared were evaluated in the same manner as described in Examples 4 to 6. As a result, the conjugates were found to exhibit specific reactivity with the CAPRIN-1 protein and CAPRIN-1-expressing cancer cells, internalization therein, and antitumor activity thereon.

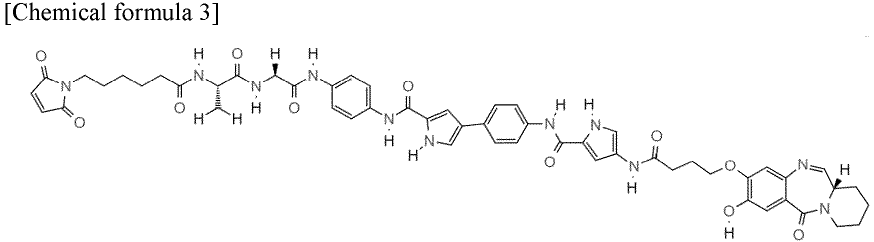

### (Example 15) Antitumor effect of conjugate of anti-CAPRIN-1 antibody and PDD derivative on cancer-bearing mice

Conjugates 160 and 166 prepared in Example 12 were evaluated for their *in vivo* antitumor effects on cancer-bearing mice. The conjugates of the present invention were examined for their antitumor effects using NOD-SCID mice in which human-derived cancer cells expressing the CAPRIN-1 protein were transplanted. Human head and neck cancer cells FaDu were mixed with Matrigel (Sigma) and subcutaneously transplanted at 10⁷ cells/mouse to the mice, which were then grown until tumor became 100 to 200 mm³ or larger to prepare cancer-bearing mice. FaDu expresses the CAPRIN-1 protein on the cell membrane surface, and Conjugate 160 and Conjugate 166 each bind to FaDu. Conjugates 160 and 166 were each administered at 6 mg/kg and 12 mg/kg, respectively, to the tail veins of FaDu-bearing mice. The administration was carried out once. PBS(-) was administered to the cancer-bearing mice, for a negative control.

The tumor sizes of the cancer-bearing mice after the administration were measured using calipers over time, and tumor volumes were calculated according to an ordinary method based on the expression: (Length of the major axis of tumor) x (Length of the minor axis of tumor)² x 0.5. As a result, the mice given Conjugates 160 and 166 at 6 mg/kg had less than 50% tumor volume 30 days after the start of the administration relative to the tumor volume of the negative control (100%). The cancer-bearing mice given Conjugates 160 and 166 at 12 mg/kg had less than 30% tumor volume.

The results of evaluation demonstrate that the conjugate of an antibody against the CAPRIN-1 protein and a PDD derivative prepared in Example 12 exhibits significantly higher antitumor effects than those of negative controls (a no-treatment control group).

## Claims

1. A conjugate of an antibody or a fragment thereof linked to benzodiazepine, wherein the antibody or the fragment thereof has immunological reactivity with a CAPRIN-1 protein having an amino acid sequence represented by any of even-numbered SEQ ID NOs among SEQ ID NOs: 2 to 30 or an amino acid sequence having 80% or more sequence identity to the amino acid sequence.

2. The conjugate according to claim 1, wherein the antibody or the fragment thereof has immunological reactivity with a partial polypeptide of the CAPRIN-1 protein, wherein the partial polypeptide has an amino acid sequence represented by any of SEQ ID NOs: 31 to 35 and 296 to 299, 308, and 309 or an amino acid sequence having 80% or more sequence identity to the amino acid sequence.

3. The conjugate according to claim 1 or 2, wherein the antibody is a monoclonal antibody or a polyclonal antibody.

4. The conjugate according to any one of claims 1 to 3, wherein the antibody or the fragment thereof is any of the following (A) to (M):
(A) an antibody or a fragment thereof, which comprises a heavy chain variable region comprising complementarity-determining regions of SEQ ID NOs: 36, 37, and 38 (CDR1, CDR2, and CDR3, respectively) and a light chain variable region comprising complementarity-determining regions of SEQ ID NOs: 40, 41, and 42 (CDR1, CDR2, and CD3, respectively) and has immunological reactivity with the CAPRIN-1 protein,
(B) an antibody or a fragment thereof, which comprises a heavy chain variable region comprising complementarity-determining regions of SEQ ID NOs: 44, 45, and 46 (CDR1, CDR2, and CDR3, respectively) and a light chain variable region comprising complementarity-determining regions of SEQ ID NOs: 48, 49, and 50 (CDR1, CDR2, and CDR3, respectively) and has immunological reactivity with the CAPRIN-1 protein,
(C) an antibody or a fragment thereof, which comprises a heavy chain variable region comprising complementarity-determining regions of SEQ ID NOs: 52, 53, and 54 (CDR1, CDR2, and CDR3, respectively) and a light chain variable region comprising complementarity-determining regions of SEQ ID NOs: 56, 57, and 58 (CDR1, CDR2, and CDR3, respectively) and has immunological reactivity with the CAPRIN-1 protein,
(D) an antibody or a fragment thereof, which comprises a heavy chain variable region comprising complementarity-determining regions of SEQ ID NOs: 60, 61, and 62 (CDR1, CDR2, and CDR3, respectively) and a light chain variable region comprising complementarity-determining regions of SEQ ID NOs: 64, 65, and 66 (CDR1, CDR2, and CDR3, respectively) and has immunological reactivity with the CAPRIN-1 protein,
(E) an antibody or a fragment thereof, which comprises a heavy chain variable region comprising complementarity-determining regions of SEQ ID NOs: 170, 171, and 172 (CDR1, CDR2, and CDR3, respectively) and a light chain variable region comprising complementarity-determining regions of SEQ ID NOs: 173, 174, and 175 (CDR1, CDR2, and CDR3, respectively) and has immunological reactivity with the CAPRIN-1 protein,
(F) an antibody or a fragment thereof, which comprises a heavy chain variable region comprising complementarity-determining regions of SEQ ID NOs: 176, 177, and 178 (CDR1, CDR2, and CDR3, respectively) and a light chain variable region comprising complementarity-determining regions of SEQ ID NOs: 179, 180, and 181 (CDR1, CDR2, and CDR3, respectively) and has immunological reactivity with the CAPRIN-1 protein,
(G) an antibody or a fragment thereof, which comprises a heavy chain variable region comprising complementarity-determining regions of SEQ ID NOs: 182, 183, and 184 (CDR1, CDR2, and CDR3, respectively) and a light chain variable region comprising complementarity-determining regions of SEQ ID NOs: 185, 186, and 187 (CDR1, CDR2, and CDR3, respectively) and has immunological reactivity with the CAPRIN-1 protein,
(H) an antibody or a fragment thereof, which comprises a heavy chain variable region comprising complementarity-determining regions of SEQ ID NOs: 188, 189, and 190 (CDR1, CDR2, and CDR3, respectively) and a light chain variable region comprising complementarity-determining regions of SEQ ID NOs: 191, 192, and 193 (CDR1, CDR2, and CDR3, respectively) and has immunological reactivity with the CAPRIN-1 protein,
(I) an antibody or a fragment thereof, which comprises a heavy chain variable region comprising complementarity-determining regions of SEQ ID NOs: 146, 147, and 148 (CDR1, CDR2, and CDR3, respectively) and a light chain variable region comprising complementarity-determining regions of SEQ ID NOs: 149, 150, and 151 (CDR1, CDR2, and CDR3, respectively) and has immunological reactivity with the CAPRIN-1 protein,
(J) an antibody or a fragment thereof, which comprises a heavy chain variable region comprising complementarity-determining regions of SEQ ID NOs: 272, 273, and 274 (CDR1, CDR2, and CDR3, respectively) and a light chain variable region comprising complementarity-determining regions of SEQ ID NOs: 275, 276, and 277 (CDR1, CDR2, and CDR3, respectively) and has immunological reactivity with the CAPRIN-1 protein,
(K) an antibody or a fragment thereof, which comprises a heavy chain variable region comprising complementarity-determining regions of SEQ ID NOs: 290, 291, and 292 (CDR1, CDR2, and CDR3, respectively) and a light chain variable region comprising complementarity-determining regions of SEQ ID NOs: 293, 294, and 295 (CDR1, CDR2, and CDR3, respectively) and has immunological reactivity with the CAPRIN-1 protein,
(L) an antibody or a fragment thereof, which comprises a heavy chain variable region comprising complementarity-determining regions of SEQ ID NOs: 301, 302, and 303 (CDR1, CDR2, and CDR3, respectively) and a light chain variable region comprising complementarity-determining regions of SEQ ID NOs: 305, 306, and 307 (CDR1, CDR2, and CDR3, respectively) and has immunological reactivity with the CAPRIN-1 protein, and
(M) an antibody or a fragment thereof, which comprises a heavy chain variable region comprising complementarity-determining regions of SEQ ID NOs: 134, 135, and 136 (CDR1, CDR2, and CDR3, respectively) and a light chain variable region comprising complementarity-determining regions of SEQ ID NOs: 137, 138, and 139 (CDR1, CDR2, and CDR3, respectively) and has immunological reactivity with the CAPRIN-1 protein.

5. The conjugate according to any one of claims 1 to 4, wherein the antibody or the fragment thereof is any of the following (a) to (al):
(a) an antibody or a fragment thereof, comprising a heavy chain variable region comprising the amino acid sequence of SEQ ID NO: 39 and a light chain variable region comprising the amino acid sequence of SEQ ID NO: 43,
(b) an antibody or a fragment thereof, comprising a heavy chain variable region comprising the amino acid sequence of SEQ ID NO: 47 and a light chain variable region comprising the amino acid sequence of SEQ ID NO: 51,
(c) an antibody or a fragment thereof, comprising a heavy chain variable region comprising the amino acid sequence of SEQ ID NO: 55 and a light chain variable region comprising the amino acid sequence of SEQ ID NO: 59,
(d) an antibody or a fragment thereof, comprising a heavy chain variable region comprising the amino acid sequence of SEQ ID NO: 63 and a light chain variable region comprising the amino acid sequence of SEQ ID NO: 67,
(e) an antibody or a fragment thereof, comprising a heavy chain variable region comprising the amino acid sequence of SEQ ID NO: 68 and a light chain variable region comprising the amino acid sequence of SEQ ID NO: 69,
(f) an antibody or a fragment thereof, comprising a heavy chain variable region comprising the amino acid sequence of SEQ ID NO: 70 and a light chain variable region comprising the amino acid sequence of SEQ ID NO: 71,
(g) an antibody or a fragment thereof, comprising a heavy chain variable region comprising the amino acid sequence of SEQ ID NO: 72 and a light chain variable region comprising the amino acid sequence of SEQ ID NO: 73,
(h) an antibody or a fragment thereof, comprising a heavy chain variable region comprising the amino acid sequence of SEQ ID NO: 74 and a light chain variable region comprising the amino acid sequence of SEQ ID NO: 75,
(i) an antibody or a fragment thereof, comprising a heavy chain variable region comprising the amino acid sequence of SEQ ID NO: 76 and a light chain variable region comprising the amino acid sequence of SEQ ID NO: 77,
(j) an antibody or a fragment thereof, comprising a heavy chain variable region comprising the amino acid sequence of SEQ ID NO: 78 and a light chain variable region comprising the amino acid sequence of SEQ ID NO: 79,
(k) an antibody or a fragment thereof, comprising a heavy chain variable region comprising the amino acid sequence of SEQ ID NO: 80 and a light chain variable region comprising the amino acid sequence of SEQ ID NO: 81,
(l) an antibody or a fragment thereof, comprising a heavy chain variable region comprising the amino acid sequence of SEQ ID NO: 82 and a light chain variable region comprising the amino acid sequence of SEQ ID NO: 83,
(m) an antibody or a fragment thereof, comprising a heavy chain variable region comprising the amino acid sequence of SEQ ID NO: 84 and a light chain variable region comprising the amino acid sequence of SEQ ID NO: 85,
(n) an antibody or a fragment thereof, comprising a heavy chain variable region comprising the amino acid sequence of SEQ ID NO: 86 and a light chain variable region comprising the amino acid sequence of SEQ ID NO: 87,
(o) an antibody or a fragment thereof, comprising a heavy chain variable region comprising the amino acid sequence of SEQ ID NO: 88 and a light chain variable region comprising the amino acid sequence of SEQ ID NO: 89,
(p) an antibody or a fragment thereof, comprising a heavy chain variable region comprising the amino acid sequence of SEQ ID NO: 90 and a light chain variable region comprising the amino acid sequence of SEQ ID NO: 91,
(q) an antibody or a fragment thereof, comprising a heavy chain variable region comprising the amino acid sequence of SEQ ID NO: 92 and a light chain variable region comprising the amino acid sequence of SEQ ID NO: 93,
(r) an antibody or a fragment thereof, comprising a heavy chain variable region comprising the amino acid sequence of SEQ ID NO: 94 and a light chain variable region comprising the amino acid sequence of SEQ ID NO: 95,
(s) an antibody or a fragment thereof, comprising a heavy chain variable region comprising the amino acid sequence of SEQ ID NO: 96 and a light chain variable region comprising the amino acid sequence of SEQ ID NO: 97,
(t) an antibody or a fragment thereof, comprising a heavy chain variable region comprising the amino acid sequence of SEQ ID NO: 98 and a light chain variable region comprising the amino acid sequence of SEQ ID NO: 99,
(u) an antibody or a fragment thereof, comprising a heavy chain variable region comprising the amino acid sequence of SEQ ID NO: 100 and a light chain variable region comprising the amino acid sequence of SEQ ID NO: 101,
(v) an antibody or a fragment thereof, comprising a heavy chain variable region comprising the amino acid sequence of SEQ ID NO: 102 and a light chain variable region comprising the amino acid sequence of SEQ ID NO: 103,
(w) an antibody or a fragment thereof, comprising a heavy chain variable region comprising the amino acid sequence of SEQ ID NO: 104 and a light chain variable region comprising the amino acid sequence of SEQ ID NO: 105,
(x) an antibody or a fragment thereof, comprising a heavy chain variable region comprising the amino acid sequence of SEQ ID NO: 106 and a light chain variable region comprising the amino acid sequence of SEQ ID NO: 107,
(y) an antibody or a fragment thereof, comprising a heavy chain variable region comprising the amino acid sequence of SEQ ID NO: 108 and a light chain variable region comprising the amino acid sequence of SEQ ID NO: 109,
(z) an antibody or a fragment thereof, comprising a heavy chain variable region comprising the amino acid sequence of SEQ ID NO: 110 and a light chain variable region comprising the amino acid sequence of SEQ ID NO: 111,
(aa) an antibody or a fragment thereof, comprising a heavy chain variable region comprising the amino acid sequence of SEQ ID NO: 112 and a light chain variable region comprising the amino acid sequence of SEQ ID NO: 113,
(ab) an antibody or a fragment thereof, comprising a heavy chain variable region comprising the amino acid sequence of SEQ ID NO: 114 and a light chain variable region comprising the amino acid sequence of SEQ ID NO: 115,
(ac) an antibody or a fragment thereof, comprising a heavy chain variable region comprising the amino acid sequence of SEQ ID NO: 116 and a light chain variable region comprising the amino acid sequence of SEQ ID NO: 117,
(ad) an antibody or a fragment thereof, comprising a heavy chain variable region comprising the amino acid sequence of SEQ ID NO: 118 and a light chain variable region comprising the amino acid sequence of SEQ ID NO: 119,
(ae) an antibody or a fragment thereof, comprising a heavy chain variable region comprising the amino acid sequence of SEQ ID NO: 120 and a light chain variable region comprising the amino acid sequence of SEQ ID NO: 121,
(af) an antibody or a fragment thereof, comprising a heavy chain variable region comprising the amino acid sequence of SEQ ID NO: 122 and a light chain variable region comprising the amino acid sequence of SEQ ID NO: 123,
(ag) an antibody or a fragment thereof, comprising a heavy chain variable region comprising the amino acid sequence of SEQ ID NO: 124 and a light chain variable region comprising the amino acid sequence of SEQ ID NO: 125,
(ah) an antibody or a fragment thereof, comprising a heavy chain variable region comprising the amino acid sequence of SEQ ID NO: 126 and a light chain variable region comprising the amino acid sequence of SEQ ID NO: 127,
(ai) an antibody or a fragment thereof, comprising a heavy chain variable region comprising the amino acid sequence of SEQ ID NO: 128 and a light chain variable region comprising the amino acid sequence of SEQ ID NO: 129,
(aj) an antibody or a fragment thereof, comprising a heavy chain variable region comprising the amino acid sequence of SEQ ID NO: 130 and a light chain variable region comprising the amino acid sequence of SEQ ID NO: 131,
(ak) an antibody or a fragment thereof, comprising a heavy chain variable region comprising the amino acid sequence of SEQ ID NO: 132 and a light chain variable region comprising the amino acid sequence of SEQ ID NO: 133, and
(al) an antibody or a fragment thereof, comprising a heavy chain variable region comprising the amino acid sequence of SEQ ID NO: 300 and a light chain variable region comprising the amino acid sequence of SEQ ID NO: 304.

6. The conjugate according to any one of claims 1 to 5, wherein the antibody is a human antibody, a humanized antibody, a chimeric antibody, or a single-chain antibody.

7. The conjugate according to any one of claims 1 to 6, wherein the antibody or the fragment thereof is linked to benzodiazepine via a linker.

8. The conjugate according to any one of claims 1 to 7, wherein the benzodiazepine is benzodiazepine having antitumor activity.

9. The conjugate according to any one of claims 1 to 8, wherein the benzodiazepine is pyrrolobenzodiazepine (PBD), indolynobenzodiazepine (IGN), pyridinobenzodiazepine (PDD), or isoquinolidinobenzodiazepine (IQB).

10. The conjugate according to any one of claims 1 to 9, wherein the benzodiazepine is DSB-120, SJG-136 (SG2000), DC-81, DSB-120, SJG-136, SG2057, SG2202, SG2285, SGD-1882, SGD-1910, SG3199, SG3249, SG2219, IMGN779, IMGN632, (S)-N-(4-aminophenyl)-4-(4-(4-(4-((2-methoxy-12-oxo-6a,7,8,9,10,12-hexahydrobenzo[e]pyrido[1,2-a][1,4]diazepin-3-yl)oxy)butanamide)-1-methyl-1H-pyrrole-2-carboxamide)phenyl)-1-methyl-1H-pyrrole-2-carboxamide, D211, D221, D231, GWL-78, KMR-28-39, or a derivative of any thereof.

11. The conjugate according to claim 10, wherein the derivative is Tesirine (SG3249 derivative), Talirine (SGD-1910 derivative), SG3364, SG3227, SG3140 (MC-Phe-Lys-PAB-SG2057), SG3170, SG3203 (MC-Phe-Lys-PAB-SG2057), SG3231, SG3400, SG3376, DGN642, DGN549, FGX5-67, FGX-2-62, or FGX11-38.

12. A pharmaceutical composition for the treatment and/or prevention of a cancer, comprising the conjugate according to any one of claims 1 to 11 as an active ingredient.

13. The pharmaceutical composition according to claim 12, wherein the cancer is a cancer expressing a CAPRIN-1 protein on the cell membrane surface.

14. The pharmaceutical composition according to claim 12 or 13, wherein the cancer is breast cancer, kidney cancer, pancreatic cancer, colorectal cancer, lung cancer, brain tumor, stomach cancer, uterine cancer, ovary cancer, prostate cancer, bladder cancer, esophagus cancer, leukemia, lymphoma, liver cancer, gallbladder cancer, bile duct cancer, sarcoma, mastocytoma, melanoma, adrenal cortex cancer, Ewing's tumor, Hodgkin's lymphoma, mesothelioma, multiple myeloma, testicle cancer, thyroid cancer, head and neck cancer, or urothelial carcinoma.

15. A method for treating and/or preventing a cancer, comprising administering the conjugate according to any one of claims 1 to 11 or the pharmaceutical composition according to any one of claims 12 to 14 to a subject.
